# EUROPÄISCHE PATENTSCHRIFT

(11) **EP 1 886 620 B1**
(45) Veröffentlichungstag und Bekanntmachung des Hinweises auf die Patenterteilung: **02.10.2013**
(21) Anmeldenummer: 07112316.0
(22) Anmeldetag: 12.07.2007
(51) Int. Cl.: A61B 1/00, A61B 1/005, A61B 1/018

(54) **Medizinisches Instrument**
Medical instrument
Instrument médical

(30) Priorität: 10.08.2006 DE 102006000399
(43) Veröffentlichungstag der Anmeldung: 13.02.2008
(73) Patentinhaber: NOVINEON HEALTHCARE TECHNOLOGY PARTNERS GMBH, 72074 Tübingen (DE)
(72) Erfinder: Schostek, Sebastian, 72070, Tübingen (DE); Ho, Chi-Nghia, 72074, Tübingen (DE); Rieber, Fabian, 70174, Stuttgart (DE); Schurr, Marc Oliver, Prof. Dr. med., 72074, Tübingen (DE)
(74) Vertreter: Winter, Brandl, Fürniss, Hübner, Röss, Kaiser, Polte - Partnerschaft

(56) Entgegenhaltungen:
- EP-A2- 0 448 284
- WO-A-91/01772
- WO-A-03/105922
- WO-A-2004/064600
- DE-A1- 3 804 491
- DE-C1- 4 414 810
- DE-C1- 19 647 761
- JP-A- 2004 008 367
- US-A- 5 178 129
- US-A- 6 068 642
- US-A1- 2002 161 330
- US-A1- 2002 165 461
- US-A1- 2004 172 116
- US-A1- 2005 085 903
- US-A1- 2006 106 288
- US-A1- 2006 171 506

## Beschreibung

Die Endoskopie ist ein in der Medizin angewandtes Verfahren zur visuellen Darstellung unterschiedlicher innerer Regionen des menschlichen Körpers unter Zuhilfenahme eines Bildgebungssystems, welches über künstliche oder natürliche Zugangswege in den Körper eingebracht wird. Mit endoskopischen Verfahren können beispielsweise der Bauchraum Laparoskopie, das Becken Pelviskopie, Gelenke Arthroskopie, die Luftwege Bronchoskopie oder der Verdauungstrakt gastrointestinale Endoskopiezugänglich gemacht werden für visuelle Inspektion, diagnostische Untersuchungen oder operative Eingriffe. Endoskopische Verfahren sind in der Regel deutlich schonender für den Patienten als entsprechende Operationsverfahren der offenen Chirurgie, da der Zugang zum einen über natürliche Körperöffnungen z.B. Bronchoskopie, gastrointestinale Endoskopie erfolgen kann oder über verhältnismäßig kleine Einschnitte im Bereich von wenigen Millimetern bis Zentimetern, z.B. in der Laparoskopie oder Arthroskopie, ein künstlicher Zugang geschaffen werden kann. Zudem hat die Einführung endoskopischer Verfahren durch speziell entwickeltes Instrumentarium neue diagnostische und therapeutische Möglichkeiten eröffnet. Allen endoskopischen Verfahren gemein ist die Verwendung eines Kamerasystems und das Vorhandensein eines durchsichtigen Fluids im Interventionsraum, z.B. Luft bzw. Stickstoff oder Kohlendioxid bei der Laparoskopie, der Bronchoskopie und der gastrointestinalen Endoskopie oder Wasser bei der Arthroskopie, mit dem das Volumen des Interventionsraums offen gehalten wird.

Der Zugang über kleine Einschnitte bzw. natürliche Körperöffnungen bringt im Regelfall eine drastische Einschränkung der Freiheitsgrade der eingeführten Instrumente mit sich, beschränkt das sensorische Feedback auf ein zweidimensionales Videobild und erhöht damit die Anforderungen an die abstraktiven und koordinativen Fähigkeiten des Operateurs. Die Entwicklung endoskopischer Verfahren basiert daher im Allgemeinen auf der Entwicklung spezialisierter Instrumente, welche die durch diese Umstände bedingten technischen Einschränkungen mittels verschiedener Verfahren, wie z.B. angepasster Bedienmöglichkeiten oder spezieller Funktionen der Instrumente zumindest teilweise kompensieren.

Bei perkutanen endoskopischen Verfahren, bei denen die Instrumente durch kleine Einschnitte in den Körper geführt werden, wie beispielsweise in der Laparoskopie oder der Arthroskopie, kann die Position der Einschnitte innerhalb der anatomisch gegebenen Grenzen weitgehend frei gewählt werden, wodurch eine Heranführung von Instrumenten an den Interventionsort aus unterschiedlichen Winkeln ermöglicht wird. Im Falle von endoluminalen endoskopischen Verfahren, die einen natürlichen Zugangsweg nutzen und die in ein tubuläres bzw. tubusähnliches Organ eingebracht werden, wie beispielsweise in der gastrointestinalen Endoskopie und der Bronchoskopie, werden Instrumente weitgehend parallel zur optischen Achse geführt. Dadurch sind im Vergleich zu perkutanen Verfahren bei endoluminalen endoskopischen Verfahren die Freiheitsgrade der Instrumente noch weiter eingeschränkt.

Des Weiteren kommen insbesondere in der gastrointestinalen Endoskopie sowie der Bronchoskopie flexible Instrumentensysteme zum Einsatz, um der Anatomie verzweigter, z.B. Bronchialsystem, oder gebogener bzw. gewundener Organe, z.B. Darm, folgen zu können. Derartige flexible Endoskope können mehr als zwei Meter lang sein. Die Endoskopspitze ist von außerhalb abwinkelbar und weist ein Kamerasystem oder ein optisches System mit nachgeschaltetem Bildleiter auf. In der Praxis verwendete Endoskope weisen oftmals einen oder zwei Arbeitskanäle auf, durch die flexible Instrumente, wie beispielsweise Fasszangen, Biopsiezangen, Schlingen oder Schneidevorrichtungen, aus der Endoskopspitze herausgeführt werden können. Durch Ausrichtung der Endoskopspitze kann unter visueller Kontrolle die Instrumentenspitze an Zielgewebe manövriert werden. Die Kraftübertragung auf die an der Endoskopspitze herausgeführten chirurgischen Instrumente ist aufgrund des flexiblen Schaftes und der großen Länge in solchen Fällen stark eingeschränkt.

Mit heute üblichen endoluminalen endoskopischen Verfahren sind durch Einsatz unterschiedlicher spezieller Instrumente verschiedene diagnostische bzw. therapeutische Verfahren möglich. Bei der gastrointestinalen Endoskopie werden heute gezielte Gewebeproben gewonnen, gestielte Polypen durch einfache Schlingenresektion abgetragen, Blutungen verödet oder gestillt, Fremdkörper entfernt und Stents platziert. Speziell für die gastrointestinale Endoskopie wurden in den letzten Jahren neue Verfahren entwickelt, um immer anspruchsvollere Aufgaben zu erfüllen. So ist es heute durch den Einsatz spezieller Instrumente möglich, im Magen oder Dickdarm über große Areale die obere Schleimhautschicht Mukosa an einem Stück zu entfernen. Bei diesem Verfahren der endoskopischen Submukosa-Dissektion, ESD, wird schrittweise die Mukosa von der darunter liegenden Schicht Submukosa abgetrennt und entfernt.

Speziell bei solchen anspruchsvollen Verfahren wie der ESD werden die gegebenen Einschränkungen der Freiheitsgrade der verwendeten Instrumente deutlich. Die Ausrichtung erfolgt über die Steuerung der Abwinkelung des flexiblen Endoskops. Die Drehung des Instruments ist häufig schwierig aufgrund der Länge und der Flexibilität des Arbeitskanals. Die einzige echte Steuerungsmöglichkeit des Instruments ist damit der Vorschub durch den Arbeitskanal. Ein weiterer Nachteil dieses Verfahrens ist, dass die Achse des Instruments an die optische Achse des Kamerasystems gebunden ist, und damit die Perspektive auf das Instrument nicht verändert werden kann.

Zur Lösung dieser Problematik wurden verschiedene Instrumentensysteme entwickelt, bei denen Instrumente zum Einsatz kommen, deren Spitze unabhängig vom Endoskop steuerbar ist. Auch Ansätze, bei denen Instrumente über Arbeitskanäle zum Interventionsort geführt werden, die außerhalb des Endoskops verlaufen und deren distale Mündungsöffnungen steuerbar sind WO 2004/064600, US 6,352,503, sind bekannt. Solche Systeme ermöglichen eine Erweiterung der Freiheitsgrade und damit die weitgehend von der Endoskopspitze unabhängige Durchführung komplexer Manöver. Zudem können zwei oder mehr Instrumente gezielt zusammenwirken, z.B. ist es möglich, mit einem Instrument Gewebe zu halten und unter Spannung zu setzen, während das andere Instrument das Gewebe gezielt durchtrennt.

Es gibt zahlreiche verschiedene Ansätze zur Aktuierung endoskopischer Instrumente für endoluminale Verfahren. Das zentrale Element solcher Entwicklungen ist zumeist ein Mechanismus zu Beugung der Instrumentenspitze. Viele dieser Mechanismen lassen aufgrund ihrer Kinematik eine direkte, intuitive mechanische Steuerung über einen mechanisch verbundenen Griff nicht zu. Dort müssen rechnergestütze Steuerungssysteme die Eingabebefehle in Steuerbefehle derart umrechnen, dass eine intuitive Steuerung der Instrumentenspitze möglich wird.Die WO 2004/064600 A2 offenbart ein Endoskop mit einem Hauptkörper, der ein proximales und ein distales Ende hat. Der Hauptkörper besteht aus einem Schaftabschnitt, an dessen proximalem Ende eine Handhabe und an dessen distalem Ende eine Instrumentenspitze angeordnet ist. Im Hauptkörper ist ferner ein Führungslumen ausgeformt, in das auch ein Instrumentenarm einführbar ist. Der aus der WO 2004/064600 A2 bekannte Arm hat einen Schaft, an dem ein distaler, manuell abkrümmbarer Endabschnitt ausgeformt ist, bestehend aus einer Anzahl seriell angeordneter Gelenkglieder, die über einen dezentral platzierten Seilzug relativ zueinander in eine Richtung verschwenkbar sind, um so den distalen Endabschnitt abzukrümmen. Der Seilzug ist innerhalb des Schafts zu einem proximalen Griffstück zurückgeführt und dort an einen Betätigungshebel angeschlossen. Wird also der Betätigungshebel verdreht, wird eine Zugkraft auf den Seilzug aufgebracht und auf den distalen Endabschnitt übertragen, der sich dementsprechend abkrümmt. Die JP 2004/008367 A offenbart ferner ein Behandlungsinstrument in Form einer Instrumenten-Einführhilfe der einschlägigen Gattung. Die aus diesem Stand der Technik bekannte Einführhilfe hat einen flexiblen Schaftabschnitt oder Rohr, an dessen proximalem Ende ein Instrumentengriff angeordnet ist und dessen distaler Endabschnitt zu einem manuell abkrümmbaren Biegeabschnitt ausgeformt ist. Der Biegeabschnitt wird dabei abgekrümmt durch Verschieben eines Betätigungsbauteils in Axialrichtung des Griffs, wobei das Betätigungsbauteil als in dem Griff 30 angeordnet definiert ist. Zur Kraftübertragung ist zwischen dem Betätigungsbauteil des Griffs und dem Biegeabschnitt ein Zugseil vorgesehen, das innerhalb des Schafts in einem darin ausgeformten Kanal geführt ist. Die Schrift US 2006/0106288 A1 offenbart den gegenstand der Präambel des Anspruchs 1.

Die Aufgabe der Erfindung besteht demzufolge darin, ein Instrumentensystem dieser Gattung zuschaffen, dessen Handhabung bei geringerem steuerungstechnischen Aufwand verbessert werden kann.

Diese Aufgabe wird durch ein Instrumentensystem mit den Merkmalen des Patentanspruchs 1 gelöst. Weitere vorteilhafte Ausgestaltungen der Erfindung sind Gegenstand der Unteransprüche.

Der Gegenstand dieser Erfindung ist eine Einführilfe bestehend aus mindestens einem schaftartigen Instrument mit einem abwinkelbaren distalen Ende und einem vorzugsweise an die menschliche Hand angepassten Griff zur manuellen Steuerung des abwinkelbaren Endes des Instruments.

Durch die spezielle Ausgestaltung des in mindestens eine Vorzugsrichtung abwinkelbaren Instruments in Verbindung mit dem angepassten Griff ist eine intuitive, direkte manuelle Steuerung der Instrumentenspitze möglich. Der Instrumentenschaft ist achsensymmetrisch ausgestaltet, sodass keine Vorzugsbiegerichtung des Instrumentenschaftes vorhanden ist. Eine Drehung des Instruments in gebogenem Zustand ist damit unabhängig vom eingestellten Drehwinkel. Der Overtube weist an seinem distalen Ende eine kappenförmige Verbindungsbrücke auf, welche einzelne Kanäle des Overtube, in welche die Instrumente und/oder das Kamerasystem einführbar sind, miteinander endseitig verbinden und an die ein wellen- oder kabelartiges Betätigungselement befestigt ist, mit dem die Rotation und der Vorschub der distalen Verbindungsbrücke des Overtube vom proximalen, extrakorporalen Ende des Overtube aus steuerbar sind. Die Instrumentenkanäle sind weitgehend mechanisch von diesem Element entkoppelt. Diese Maßnahme ermöglicht eine gute Steuerung des Overtube bei hoher Flexibilität, da beispielsweise bei Abkrümmen des Overtube keine Stauchung/Dehnung der Instrumentenkanäle erfolgt, was eine einfache und schonende Einführung des Systems in den menschlichen Körper begünstigt.

Die Erfindung wird nachstehend anhand bevorzugter Ausführungsbeispiele unter Bezugnahme auf die begleitenden Zeichnungen näher erläutert.

Die Fig. 1 bis 18d zeigen Varianten eines einzuführenden Instruments vorliegend in Form eines Endoskops oder Trokars gemäß der vorliegenden Erfindung;

Die Fig. 19 bis 25 zeigen das einzuführende Instrument in Kombination mit einem in das Instrument eingeführten chirurgischen Instrument sowie einer Betätigungsvorrichtung zur Abkrümmung des Instruments und zur Handhabung des chirurgischen Instruments;

Die Fig. 26 bis 29 zeigen die prinzipielle Ausgestaltung eines Overtube;

Die Fig. 30 bis 38b zeigen Varianten der Betätigungsvorrichtung;

Die Fig. 39 bis 51b zeigen Varianten des Overtube.

### Instrument

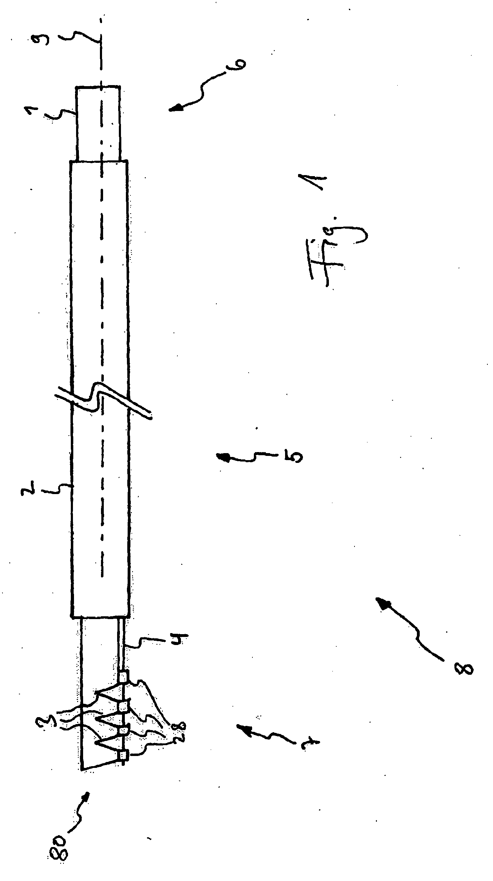

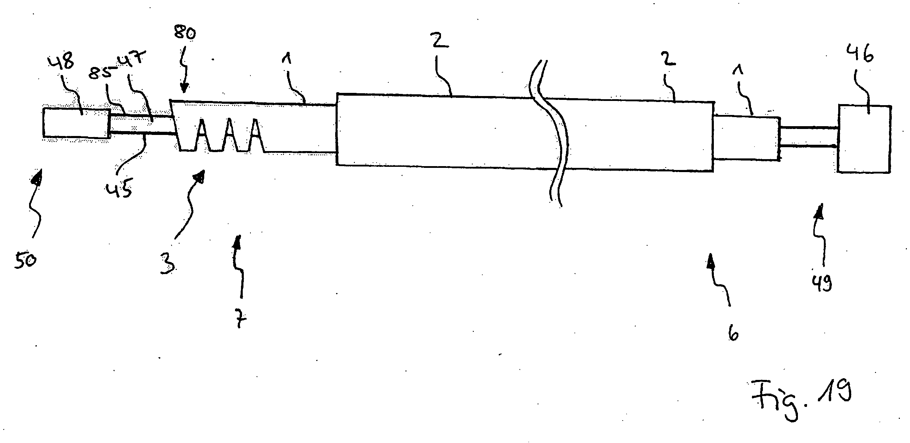

Das Instrument 8 gemäß einem bevorzugten Ausführungsbeispiel der Erfindung (siehe Fig. 19), welches im vorliegenden Fall in Form eines Endoskops ausgebildet ist, dient zur Ausrichtung einer zweiten Vorrichtung 45 insbesondere eines chirurgischen Instruments in zumindest eine Vorzugsrichtung 81 . Das endoskopartige Instrument 8 besteht aus einem Instrumentenschaft 5 mit einem distalen Ende 7 , einem proximalen Ende 6 . Das distale Ende 7 weist erste, vorzugsweise scharnierartige Strukturen 3 (sog. Sollbiegestellen) auf, die eine Abwinkelung einer distalen Instrumentenspitze 80 in die zumindest eine Vorzugsrichtung 81 begünstigen. Das Instrument 8 weist des Weiteren ein Zugelement 4 auf, durch dessen Betätigung die Abwinkelung der Instrumentenspitze 80 bewirkt werden kann. Der Instrumentenschaft 5 weist ein inneres Rohrelement 1 und ein äußeres Rohrelement 2 auf, wobei das innere Rohrelement 1 in dem äußeren Rohrelement 2 geführt wird. Das innere Rohrelement 1 schaut am distalen Ende 7 des Instruments 8 aus dem äußeren Rohrelement 2 heraus und weist im herausragenden Abschnitt die ersten Strukturen 3 auf. Das äußere Rohrelement 2 ist parallel zur Instrumentenachse 9 relativ zum inneren Rohrelement 1 verschiebbar und ist mit dem Zugelement 4 verbunden (siehe Fig. 1). Bei Verschieben des äußeren Rohrelements 2 parallel zur Instrumentenachse 9 in Richtung des proximalen Endes 6 des Instruments 8 wird das Zugelement 4 betätigt und damit im Bereich der ersten Strukturen 3 eine Abwinkelung der Instrumentenspitze 80 in die Vorzugsrichtung 81 bewirkt. Des Weiteren kann bei entsprechender Ausgestaltung des Zugelements 4 bei Verschieben des äußeren Rohrelements 2 parallel zur Instrumentenachse 9 in Richtung des distalen Endes 7 des Instruments 8 eine Abwinkelung der Instrumentenspitze 80 entgegen der Vorzugsbiegerichtung bewirkt werden. Durch die achsensymmetrische Gestaltung des Instruments 8 im Bereich des Instrumentenschaftes 5 wird eine Vorzugsbiegerichtung des Instrumentenschaftes 5 vermieden. Bei Rotation des Instruments 8 in gebogenem Zustand ist damit das benötigte Drehmoment unabhängig vom eingestellten Biegewinkel.

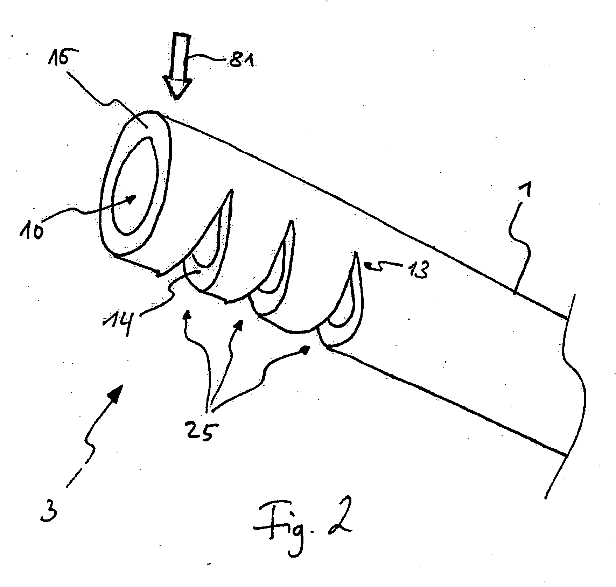

Die ersten Strukturen 3 dienen zur Erzeugung einer Vorzugsbiegerichtung 81 in einem begrenzten Abschnitt des inneren Rohrelements 1 am distalen Ende 7 des Instruments 8 . Dies wird erreicht durch Freiräume oder Einkerbungen 25 am inneren Rohrelement 1 , die sich im Längsabstand des inneren Rohrelements 1 aneinander reihen. Die Einkerbungen 25 weisen jeweils gegenüberliegende Flanken 14 auf, die sich bei Abwinkelung der Instrumentenspitze 80 annähern. Die ersten Strukturen 3 sind dabei vorzugsweise derart gestaltet, dass die Axialbohrung des inneren Rohrelements 1 nicht eingeschränkt wird (siehe Fig. 2).

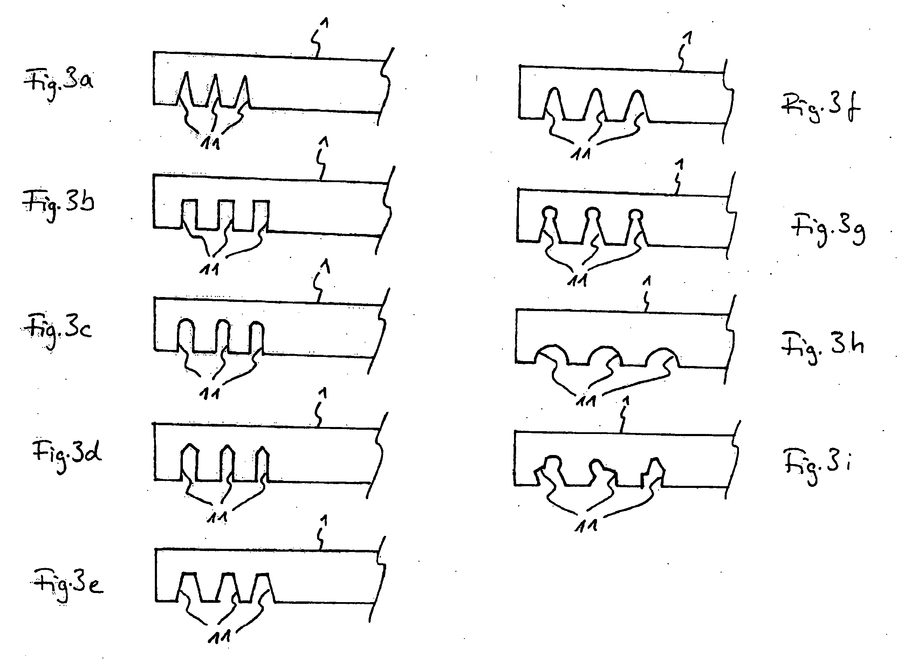

Ein Beispiel für die Ausgestaltung der ersten Strukturen 3 ist die Erzeugung von seitlichen Aussparungen 11 am inneren Rohrelement 1 am distalen Ende 7 des Instruments 8 , wie sie in unterschiedlichen Formen in den Fig. 3a bis 3i dargestellt sind.

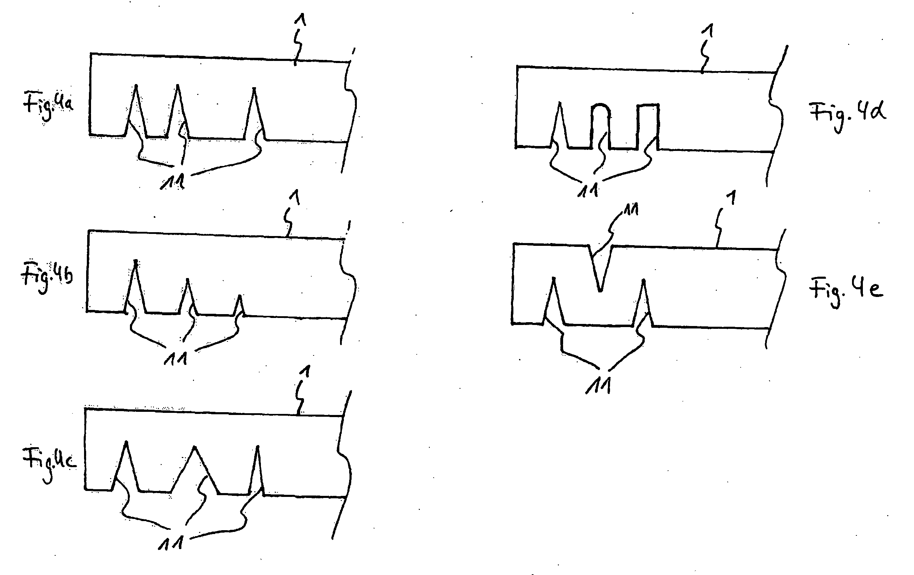

In einer vorteilhaften Ausgestaltung der seitlichen Aussparungen 11 weist diese einen dreieckigen Querschnitt auf siehe Fig. 3a . In einer weiteren vorteilhaften Ausgestaltung der seitlichen Aussparungen 11 weist diese einen rechteckigen Querschnitt auf (siehe Fig. 3b). In einer weiteren vorteilhaften Ausgestaltung der seitlichen Aussparungen 11 weist diese einen solchen Querschnitt auf, wobei die Aussparungsflanken 14 parallel verlaufen und der Aussparungsgrund 13 jeweils abgerundet ist (siehe Fig. 3c). In einer weiteren vorteilhaften Ausgestaltung der seitlichen Aussparungen 11 weist diese einen solchen Querschnitt auf, wobei die Aussparungsflanken 14 parallel verlaufen und der Aussparungsgrund 13 V-förmig gestaltet ist (siehe Fig. 3d). In einer weiteren vorteilhaften Ausgestaltung der seitlichen Aussparungen 11 weist die seitliche Aussparung 11 einen trapezförmigen Querschnitt auf (siehe Fig. 3e). In einer weiteren vorteilhaften Ausgestaltung der seitlichen Aussparungen 11 weist die seitliche Aussparung 11 einen dreieckigen Querschnitt auf, wobei der Aussparungsgrund eine Rundung aufweist (siehe Fig. 3f und Fig. 3g). Durch diese Rundung können lokale Spannungsüberhöhungen im Bereich der Spitze des Dreiecks bei Abwinkelung der Instrumentenspitze 80 reduziert werden. In einer weiteren vorteilhaften Ausgestaltung der seitlichen Aussparungen 11 weist diese einen halbkreisförmigen Querschnitt auf (siehe Fig. 3h). In einer weiteren vorteilhaften Ausgestaltung der seitlichen Aussparungen 11 weist diese einen unregelmäßigen Querschnitt auf (siehe Fig. 3i). In einer weiteren vorteilhaften Ausgestaltung der seitlichen Aussparungen 11 sind die seitlichen Aussparungen 11 in regelmäßigen Abständen zueinander angeordnet (siehe Fig. 3a). In einer weiteren vorteilhaften Ausgestaltung der seitlichen Aussparungen 11 sind die seitlichen Aussparungen 11 in unterschiedlichen Abständen zueinander angeordnet (siehe Fig. 4a). In einer weiteren vorteilhaften Ausgestaltung der seitlichen Aussparungen 11 weisen die seitlichen Aussparungen 11 jeweils dieselbe Tiefe auf (siehe Fig. 3a). In einer weiteren vorteilhaften Ausgestaltung der seitlichen Aussparungen 11 weisen die seitlichen Aussparungen 11 jeweils unterschiedliche Tiefen auf (siehe Fig. 4b). In einer weiteren vorteilhaften Ausgestaltung der seitlichen Aussparungen 11 weisen die seitlichen Aussparungen 11 jeweils dieselbe Breite auf (siehe Fig. 3a). In einer weiteren vorteilhaften Ausgestaltung der seitlichen Aussparungen 11 weisen die seitlichen Aussparungen 11 jeweils unterschiedliche Breiten auf (siehe Fig. 4c). In einer weiteren vorteilhaften Weiterbildung der seitlichen Aussparungen 11 weisen diese jeweils dieselbe Form auf (siehe Fig. 3a). In einer weiteren vorteilhaften Ausgestaltung der seitlichen Aussparungen 11 weisen diese jeweils unterschiedliche Formen auf (siehe Fig. 4d). In einer weiteren vorteilhaften Ausgestaltung der seitlichen Aussparungen 11 sind die seitlichen Aussparungen 11 jeweils in dieselbe Richtung, vorzugsweise die Vorzugsbiegerichtung 81 ausgerichtet (siehe Fig. 3a). In einer weiteren vorteilhaften Ausgestaltung der seitlichen Aussparungen 11 sind die seitlichen Aussparungen 11 jeweils in unterschiedliche Richtungen ausgerichtet (siehe Fig. 4e).

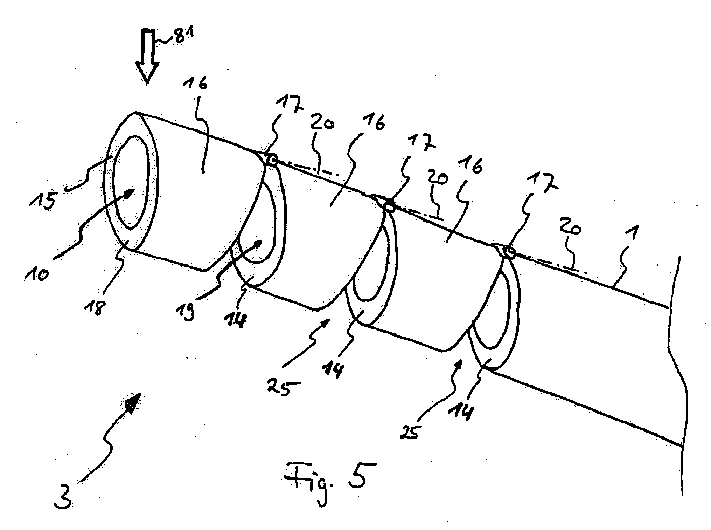

Ein weiteres Beispiel für die Ausgestaltung der ersten Strukturen 3 sieht eine Verbindung einzelner Segmente 16 des inneren Rohrelements 1 über externe Scharnierelemente 17 vor. Die einzelnen Rohrsegmente 16 werden vorzugsweise derart angeordnet, dass eine fortlaufende Kette von untereinander über die Scharnierelemente 17 verbundenen Segmenten 16 entsteht. Diese Rohrsegmente 16 lassen sich jeweils relativ zum benachbarten Segment 16 um die Schwenkachse 20 des Scharnierelements 17 , das sich zwischen den jeweiligen benachbarten Segmenten 16 befindet, kippen bzw. abwinkeln. Die Segmente 16 weisen jeweils eine durchgehende Axialbohrung 19 auf (siehe Fig. 5).

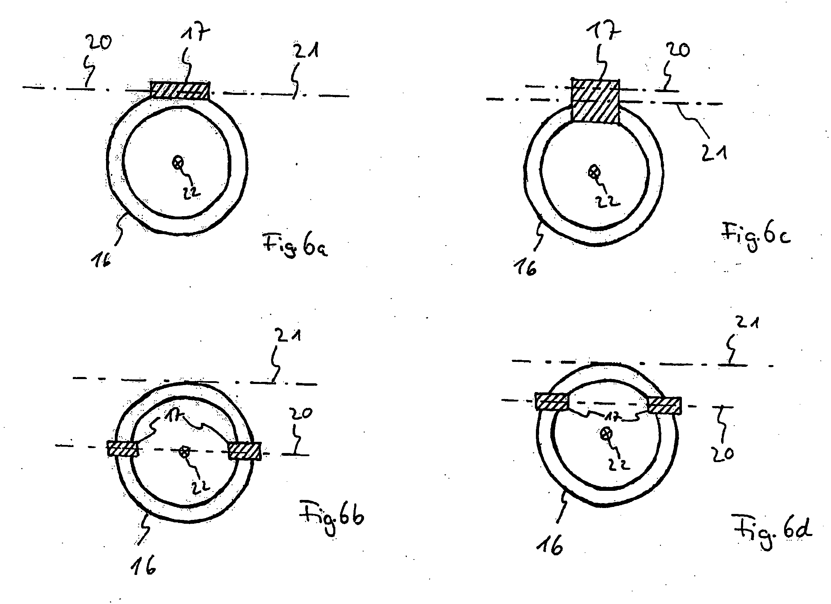

In einer vorteilhaften Ausgestaltung des Scharnierelements 17 ist das Scharnierelement 17 derart mit einem Segment 16 verbunden, dass die Schwenkachse 20 des Scharnierelements 17 kolinear mit eine Linie 21 tangential zur Außenseite des Segments verläuft (siehe Fig. 6a). In einer weiteren vorteilhaften Ausgestaltung des Scharnierelements 17 ist dieses derart mit einem Segment 16 verbunden, dass die Schwenkachse 20 des Scharnierelements 17 die Achse 22 des Segments 16 kreuzt (siehe Fig. 6b). In einer weiteren vorteilhaften Ausgestaltung des Scharnierelements 17 ist das Scharnierelement 17 derart mit einem Segment 16 verbunden, dass die Schwenkachse 20 des Scharnierelements 17 zwischen einer Linie 21 tangential zur Außenseite des Segments und der Achse 22 des Segments verläuft (siehe Fig. 6c). In einer weiteren vorteilhaften Ausgestaltung des Scharnierelements 17 ist das Scharnierelement 17 derart mit einem Segment 16 verbunden, dass die Schwenkachse 20 des Scharnierelements 17 außerhalb des Segments verläuft (siehe Fig. 6d).

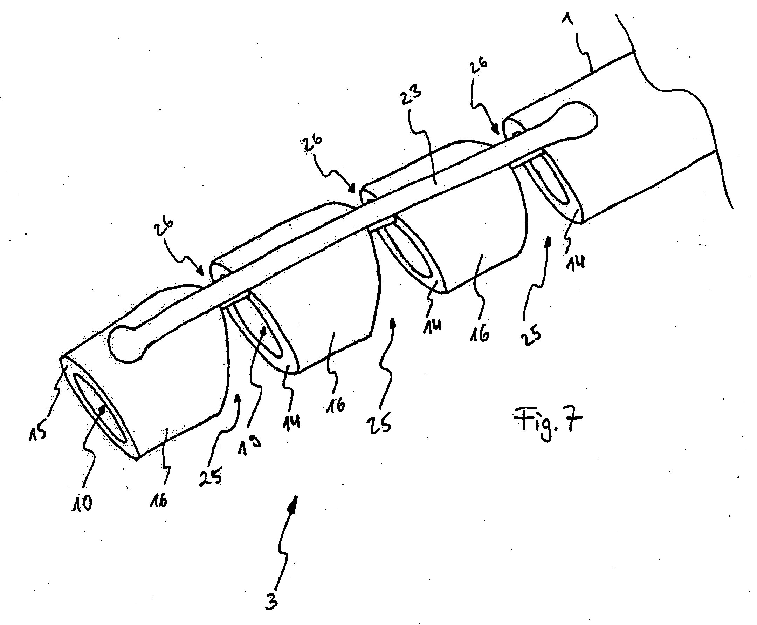

Ein weiteres Beispiel für die Ausgestaltung der ersten Strukturen 3 ist die Verbindung einzelner Rohrsegmente 16 über mindestens ein externes Biegeelement 23 . Die einzelnen Rohrsegmente 16 werden vorzugsweise derart angeordnet, dass eine fortlaufende Kette von untereinander über das externe Biegeelement 23 verbundenen Segmenten 16 entsteht. Diese Segmente 16 lassen sich jeweils relativ zum benachbarten Segment 16 durch Deformation des Biegeelements 23 im Biegebereich 26 kippen. Die Segmente 16 weisen jeweils eine durchgehende Axialbohrung 19 auf (siehe Fig. 7).

Das Biegeelement 23 dient zur Verformung bei einer Abwinkelung der Instrumentenspitze 80 . Andere Elemente wie beispielsweise die Segmente 16 werden daher weniger mechanisch belastet als bei der Verwendung von seitlichen Aussparungen 11 , gleichzeitig ist aber eine deformationsbedingte Rückstellkraft einfacher zu realisieren als bei der Verwendung von Scharnierelementen 17 . Durch eine gezielte Gestaltung des Biegelements 23 können die mechanischen Eigenschaften gezielt beeinflusst werden. Insbesondere die Verwendung von Formgedächtnislegierungen wie beispielsweise Nickel-Titan-Legierungen bieten sich als Werkstoff für das Biegeelement 23 an, da sie auch bei starker Verformung eine Rückkehr zum ursprünglichen Zustand gewährleisten können.

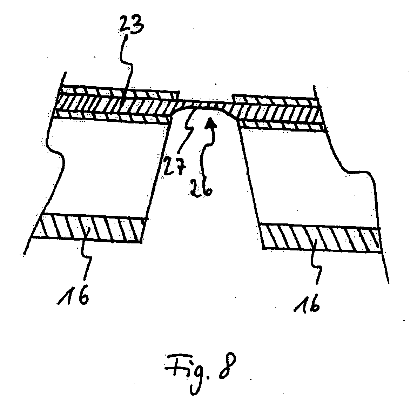

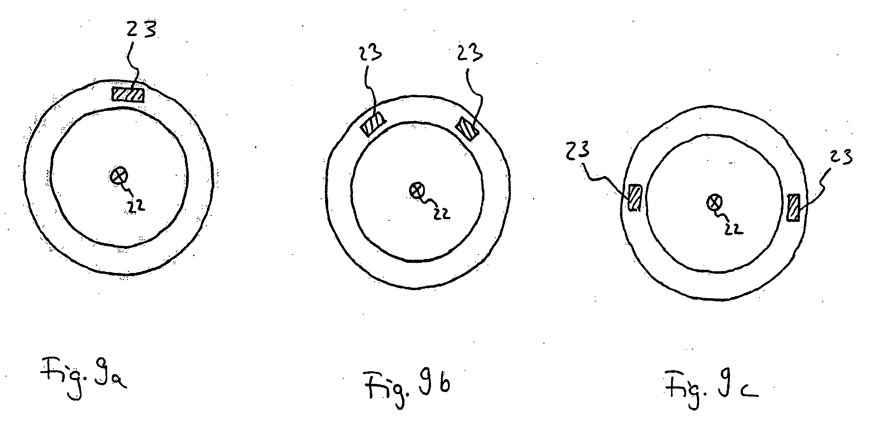

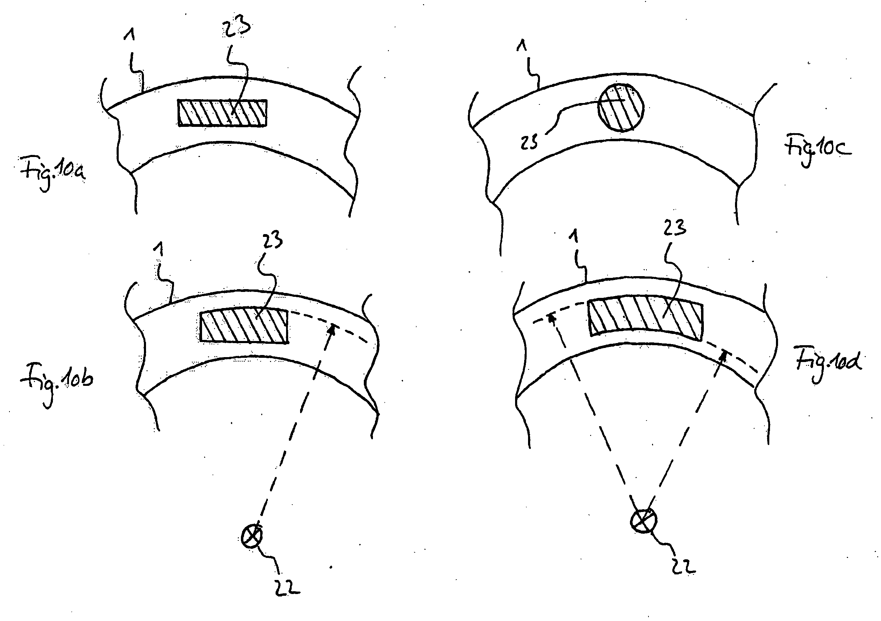

In einer vorteilhaften Ausgestaltung des Biegeelements 23 besteht das Biegeelement 23 aus einer Formgedächtnislegierung, vorzugsweise aus einer Nickel-Titan-Legierung. In einer weiteren vorteilhaften Ausgestaltung des Biegeelements 23 weist dieses im Bereich des Biegebereichs 26 eine Verjüngung 27 auf. Diese Verjüngung 27 lokalisiert die Deformation bei Abwinkelung der Instrumentenspitze 80 auf die Biegebereiche 26 des Biegeelements 23 , die vorzugsweise zwischen den Segmenten 16 positioniert sind (siehe Fig. 8). In einer weiteren vorteilhaften Ausgestaltung des Biegeelements 23 weist das Biegeelement 23 im Biegebereich 26 einen rechteckigen Querschnitt auf (siehe Fig. 10a). In einer weiteren vorteilhaften Ausgestaltung des Biegeelements 23 weist dieses im Biegebereich 26 einen solchen Querschnitt auf, der auf der Rohrsegment-Außenseite zu einer konvexen Wölbung ausgebildet ist (siehe Fig. 10b). In einer weiteren vorteilhaften Ausgestaltung des Biegeelements 23 weist das Biegeelement 23 im Biegebereich 26 einen runden Querschnitt auf (siehe Fig. 10c). In einer weiteren vorteilhaften Ausgestaltung des Biegeelements 23 weist dieses im Biegebereich 26 einen solchen Querschnitt auf, der auf der Rohrsegment-Außenseite zu einer konvexen Wölbung und auf der Rohrsegment-Innenseite zu einer konkaven Wölbung ausgeformt ist (siehe Fig. 10d). In einer weiteren vorteilhaften Ausgestaltung des Biegeelements 23 weist das Biegeelement 23 im Biegebereich 26 genau einen Steg auf (siehe Fig. 9a). In einer weiteren vorteilhaften Ausgestaltung des Biegeelements 23 weist das Biegeelement 23 im Biegebereich 26 zwei Stege auf (siehe Fig. 9b). In einer weiteren vorteilhaften Ausgestaltung des Biegeelements 23 weist das Biegeelement 23 im Biegebereich 26 zwei Stege auf, wobei sich die zwei Stege an diametral gegenüberliegenden Punkten des inneren Rohrelements 1 befinden (siehe Fig. 9c).

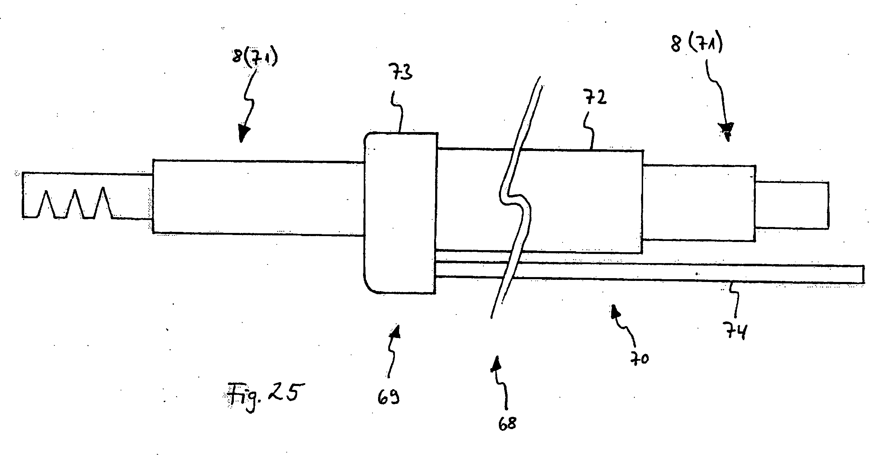

Ein weiteres Beispiel für eine Ausgestaltung der ersten Strukturen 3 ist eine Kombination eines externen Biegeelements 23 mit am inneren Rohrelement integrierten seitlichen Aussparungen 11 . Damit können durch Einbettung eines Biegeelements 23 in das innere Rohrelement und durch Erzeugen der seitlichen Aussparungen 11 mit minimaler Anzahl von Komponenten erste Strukturen 3 realisiert werden, deren mechanische Eigenschaften durch selektive Gestaltung des Biegeelements 23 eingestellt werden können (siehe Fig. 25).

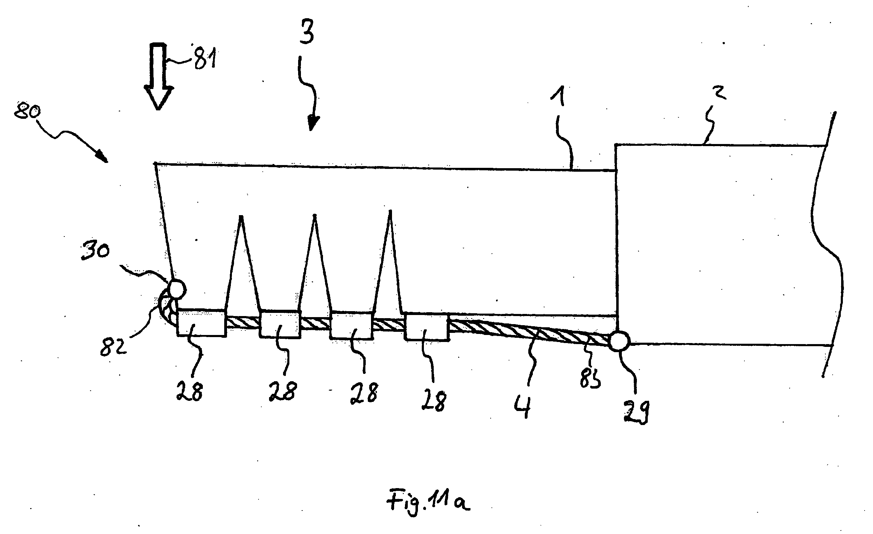

Das Zug- oder Zug-/Druckelement 4 dient zur Kraftübertragung zwischen dem äußeren Rohrelement 2 und den ersten Strukturen 3 . Das Zugelement 4 ist dazu über eine erste mechanische Verbindung 29 mit dem äußeren Rohrelement 2 und über eine zweite mechanische Verbindung 30 mit der Instrumentenspitze 80 verbunden. Die ersten Strukturen 3 befinden sich dabei vorzugsweise zwischen der ersten mechanischen Verbindung 29 und der zweiten mechanischen Verbindung 30 . Das Zugelement 4 wird dabei von der ersten mechanischen Verbindung 29 zur zweiten mechanischen Verbindung 30 an den ersten Strukturen 3 auf der Seite der Vorzugsbiegerichtung 81 vorbeigeführt. Das Zugelement 4 wird dabei von einer zweiten Zugelementführung 28 geführt, die auf der Seite der Vorzugsbiegerichtung 81 im Bereich der ersten Strukturen 3 mit dem inneren Rohrelement 1 verbunden ist (siehe Fig. 11a).

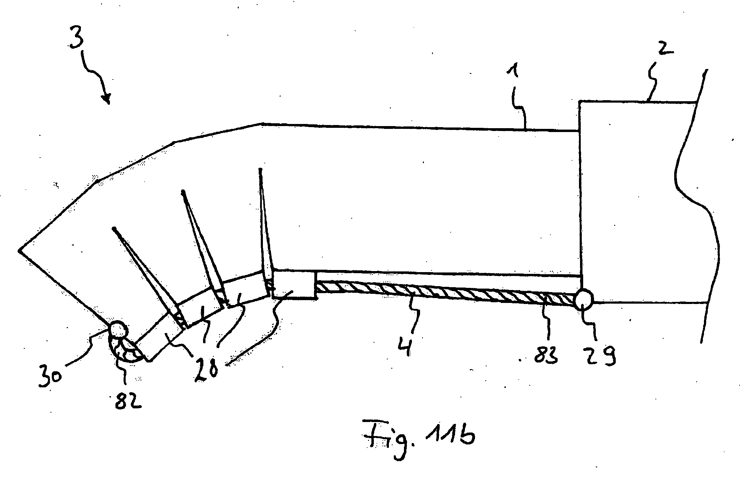

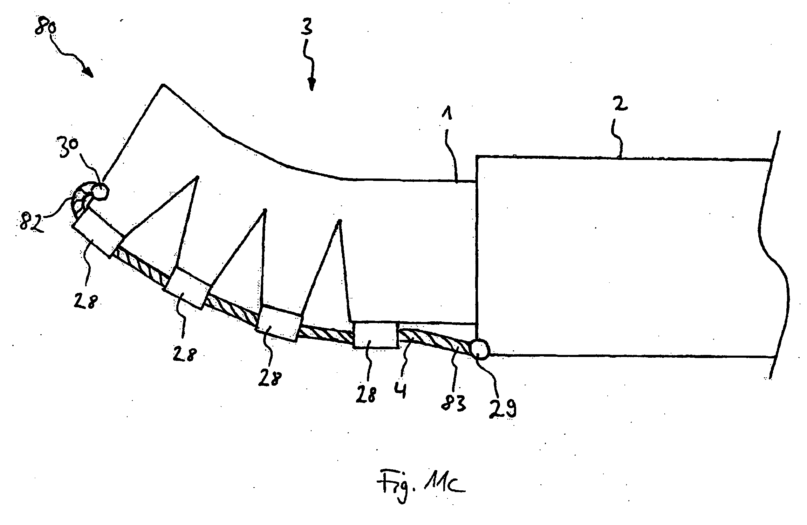

Die zweite Zugelementführung 28 ist derart gestaltet, dass bei Verschieben des äußeren Rohrelements 2 parallel zur Instrumentenachse 9 in Richtung des proximalen Endes 6 des Instruments 8 die Instrumentenspitze 80 in Richtung der Vorzugsrichtung 81 abgewinkelt wird (siehe Fig. 11b). Das Zugelement 4 und die zweite Zugelementführung 28 sind des weiteren wahlweise etwa nach dem Prinzip eines Bowdenzuges derart gestaltet, dass bei Verschieben des äußeren Rohrelements 2 parallel zur Instrumentenachse 9 in Richtung des distalen Endes 7 des Instruments 8 die Instrumentenspitze 80 entgegen der Vorzugsrichtung 81 abgewinkelt wird, (siehe Fig. 11c). Hierfür besteht die Zugelementführung aus einer Anzahl von Ösen, welche an den einzelnen Rohrsegmenten jeweils im Bereich zwischen den Einkerbungen montiert sind, derart, dass sich die Ösenlöcher im Wesentlichen in einer Linie ausrichten.

Die erste mechanische Verbindung 29 verbindet das proximale Ende 83 des Zugelements 4 derart mit dem äußeren Rohrelement 2 , dass bei einer Kraft auf das Zugelement 4 in Richtung zum distalen Ende 7 des Instruments 8 die Kraft auf das äußere Rohrelement 2 übertragen wird. Wahlweise verbindet die erste mechanische Verbindung 29 das proximale Ende 83 des Zugelements 4 derart mit dem äußeren Rohrelement 2 , dass bei einer Kraft auf das Zugelement in Richtung des proximalen Endes 6 des Instruments 8 die Kraft (Druckkraft) auf das äußere Rohrelement 2 übertragen wird.

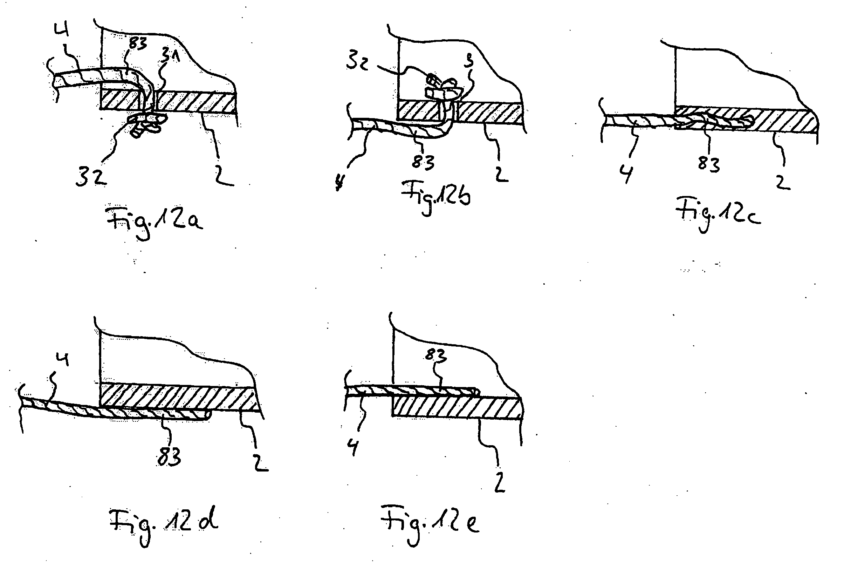

In einer vorteilhaften Ausgestaltung der ersten mechanischen Verbindung 29 ist das Zugelement 4 über eine seitliche Öffnung 31 im äußeren Rohrelement 2 geführt, wobei das Zugelement 4 eine proximale Verdickung 32 aufweist, beispielsweise ein Knoten oder ein am Zugelement 4 mechanisch befestigtes Bauteil, die das Passieren des proximalen Endes 83 des Zugelements 4 durch die seitliche Öffnung 31 im äußeren Rohrelement 2 blockiert. Die proximale Verdickung liegt hierbei auf der Außenseite des äußeren Rohrelements 2 (siehe Fig. 12a). In einer weiteren vorteilhaften Ausgestaltung der ersten mechanischen Verbindung 29 ist das Zugelement 4 über eine seitliche Öffnung 31 im äußeren Rohrelement 2 geführt, wobei das Zugelement 4 eine proximale Verdickung 32 aufweist, beispielsweise ein Knoten oder ein am Zugelement 4 mechanisch befestigtes Bauteil, die das Passieren des proximalen Endes 83 des Zugelements 4 durch die seitliche Öffnung 31 im äußeren Rohrelement 2 blockiert. Die proximale Verdickung liegt hierbei auf der Innenseite des äußeren Rohrelements 2 (siehe Fig. 12b). In einer weiteren vorteilhaften Ausgestaltung der ersten mechanischen Verbindung 29 ist das proximale Ende 83 des Zugelements 4 in die Wand des äußeren Rohrelements 2 eingelassen (siehe Fig. 12c). In einer weiteren vorteilhaften Ausgestaltung der ersten mechanischen Verbindung 29 ist das proximale Ende 83 des Zugelements 4 an der Außenseite des äußeren Rohrelements 2 mit dem äußeren Rohrelement 2 verbunden (siehe Fig. 12d). In einer weiteren vorteilhaften Ausgestaltung der ersten mechanischen Verbindung 29 ist das proximale Ende 83 des Zugelements 4 an der Innenseite des äußeren Rohrelements 2 mit dem äußeren Rohrelement 2 verbunden (siehe Fig. 12e).

Die zweite mechanische Verbindung 30 verbindet das distale Ende 82 des Zugelements 4 derart mit dem inneren Rohrelement 1 , dass bei einer Kraft auf das Zugelement 4 in Richtung des proximalen Endes 6 des Instruments 8 die Kraft auf das innere Rohrelement 1 übertragen wird. Wahlweise verbindet die zweite mechanische Verbindung 30 das distale Ende 82 des Zugelements 4 derart mit dem inneren Rohrelement 1 , dass bei einer Kraft auf das Zugelement in Richtung des distalen Endes 7 des Instruments 8 die Kraft auf das innere Rohrelement 1 übertragen wird.

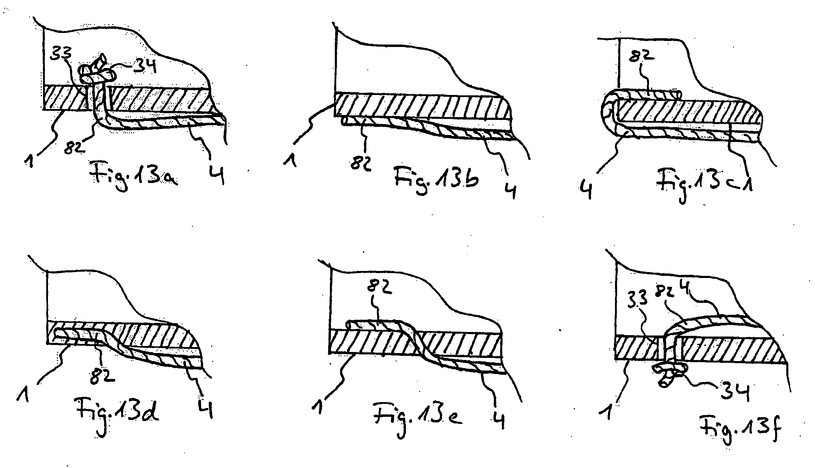

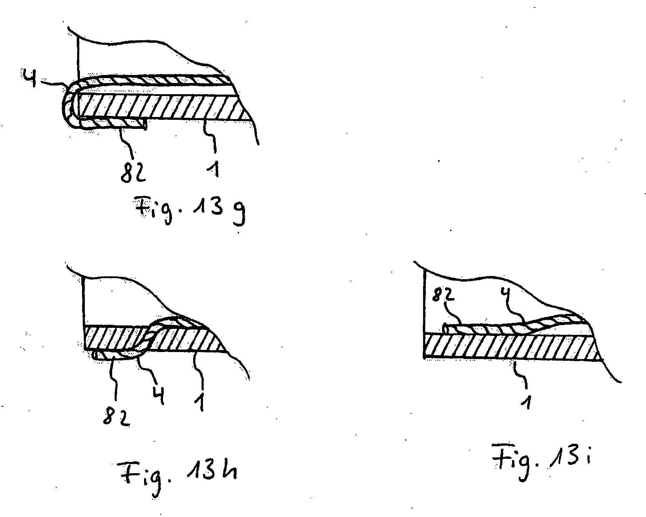

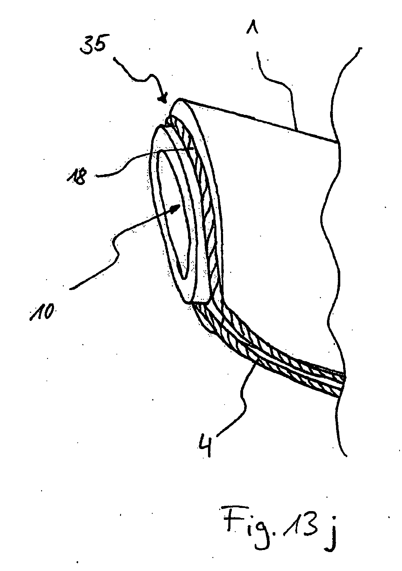

In einer vorteilhaften Ausgestaltung der zweiten mechanischen Verbindung 30 ist das Zugelement 4 über eine seitliche Öffnung 33 im inneren Rohrelement 1 geführt, wobei das Zugelement 4 eine distale Verdickung 34 aufweist, beispielsweise ein Knoten oder ein am Zugelement 4 mechanisch befestigtes Bauteil, die das Passieren des distalen Endes 82 des Zugelements 4 durch die seitliche Öffnung 33 im inneren Rohrelement 1 blockiert. Die distale Verdickung liegt hierbei auf der Innenseite des inneren Rohrelements 1 (siehe Fig. 13a). In einer weiteren vorteilhaften Ausgestaltung der zweiten mechanischen Verbindung 30 ist das Zugelement 4 über eine seitliche Öffnung 33 im inneren Rohrelement 1 geführt, wobei das Zugelement 4 eine distale Verdickung 34 aufweist, beispielsweise ein Knoten oder ein am Zugelement 4 mechanisch befestigtes Bauteil, die das Passieren des distalen Endes 82 des Zugelements 4 durch die seitliche Öffnung 33 im inneren Rohrelement 1 blockiert. Die distale Verdickung liegt hierbei auf der Außenseite des inneren Rohrelements 1 (siehe Fig. 13f). In einer weiteren vorteilhaften Ausgestaltung der zweiten mechanischen Verbindung 30 ist das distale Ende 82 des Zugelements 4 an der Außenseite des inneren Rohrelements 1 mit dem inneren Rohrelement 1 verbunden, wobei das Zugelement 4 vom proximalen Ende 6 des Instruments 8 an der Außenseite des inneren Rohrelements 1 herangeführt wird (siehe Fig. 13b). In einer weiteren vorteilhaften Ausgestaltung der zweiten mechanischen Verbindung 30 ist das distale Ende 82 des Zugelements 4 an der Außenseite des inneren Rohrelements 1 mit dem inneren Rohrelement 1 verbunden, wobei das Zugelement 4 vom proximalen Ende 6 des Instruments 8 an der Innenseite des inneren Rohrelements 1 herangeführt wird und über die Stirnfläche 15 des inneren Rohrelements 1 auf die Außenseite des inneren Rohrelements 1 geführt wird (siehe Fig. 13g). In einer weiteren vorteilhaften Ausgestaltung der zweiten mechanischen Verbindung 30 ist das distale Ende 82 des Zugelements 4 an der Außenseite des inneren Rohrelements 1 mit dem inneren Rohrelement 1 verbunden, wobei das Zugelement 4 vom proximalen Ende 6 des Instruments 8 an der Innenseite des inneren Rohrelements 1 herangeführt wird und durch die Wand des inneren Rohrelements 1 auf die Außenseite des inneren Rohrelements 1 geführt wird (siehe Fig. 13h). In einer weiteren vorteilhaften Ausgestaltung der zweiten mechanischen Verbindung 30 ist das distale Ende 82 des Zugelements 4 in die Wand des inneren Rohrelements 1 eingelassen (siehe Fig. 13d). In einer weiteren vorteilhaften Ausgestaltung der zweiten mechanischen Verbindung 30 ist das distale Ende 82 des Zugelements 4 an der Innenseite des inneren Rohrelements 1 mit dem inneren Rohrelement 1 verbunden, wobei das Zugelement 4 vom proximalen Ende 6 des Instruments 8 an der Innenseite des inneren Rohrelements 1 herangeführt wird (siehe Fig. 13i). In einer weiteren vorteilhaften Ausgestaltung der zweiten mechanischen Verbindung 30 ist das distale Ende 82 des Zugelements 4 an der Innenseite des inneren Rohrelements 1 mit dem inneren Rohrelement 1 verbunden, wobei das Zugelement 4 vom proximalen Ende 6 des Instruments 8 an der Außenseite des inneren Rohrelements 1 herangeführt wird und über die Stirnfläche 15 des inneren Rohrelements 1 auf die Innenseite des inneren Rohrelements 1 geführt wird (siehe Fig. 13c). In einer weiteren vorteilhaften Ausgestaltung der zweiten mechanischen Verbindung 30 ist das distale Ende 82 des Zugelements 4 an der Innenseite des inneren Rohrelements 1 mit dem inneren Rohrelement 1 verbunden, wobei das Zugelement 4 vom proximalen Ende 6 des Instruments 8 an der Außenseite des inneren Rohrelements 1 herangeführt wird und durch die Wand des inneren Rohrelements 1 auf die Innenseite des inneren Rohrelements 1 geführt wird (siehe Fig. 13e). In einer weiteren vorteilhaften Ausgestaltung der zweiten mechanischen Verbindung 30 weist das distale Ende 82 des Zugelements 4 eine Schlaufe 18 auf. Die Stirnfläche 15 des Inneren Rohrelements 1 weist eine erste Zugelementführung 35 auf, in der die Schlaufe 18 des Zugelements 4 geführt wird, vorzugsweise um die distale Öffnung 10 des inneren Rohrelements 1 herum (siehe Fig. 13j).

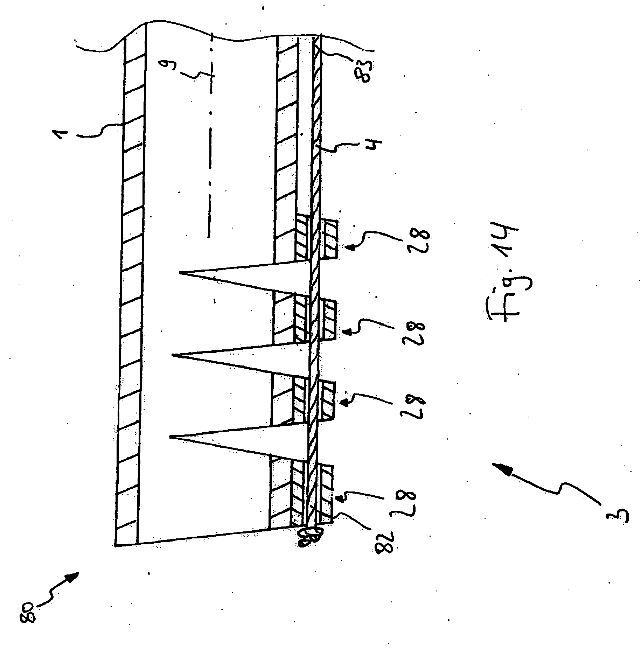

Die Abwinkelung der Instrumentenspitze 80 erfolgt über die ersten Strukturen 3 , die bei Betätigung des Zugelements 4 um eine, vorzugsweise aber um mehrere Achsen in die Vorzugsbiegerichtung 81 durch lokale Verformung des inneren Rohrelements 1 oder durch Scharnierelemente 17 abgewinkelt werden. Die zweite Zugelementführung 28 ist dabei derart gestaltet, dass eine auf das Zugelement 4 wirkende Kraft derart auf die ersten Strukturen 3 übertragen wird, dass eine gleichmäßige Abwinkelung der Instrumentenspitze 80 erfolgt. Die zweite Zugelementführung 28 ist dabei derart gestaltet, dass das Zugelement 4 der bei der Abwinkelung der Instrumentenspitze 80 erzeugten Beugung folgt (siehe Fig. 14). Demzufolge sind die zwischen den Einkerbungen an den Rohrsegmenten angebrachte Ösen 28 keilförmig oder derart dünnwandig ausgebildet, dass bei maximalem Abkrümmwinkel der ersten Strukturen die Flanken der Auskerbungen unbeeinflusst von den Ösen miteinander in Anlage stehen.

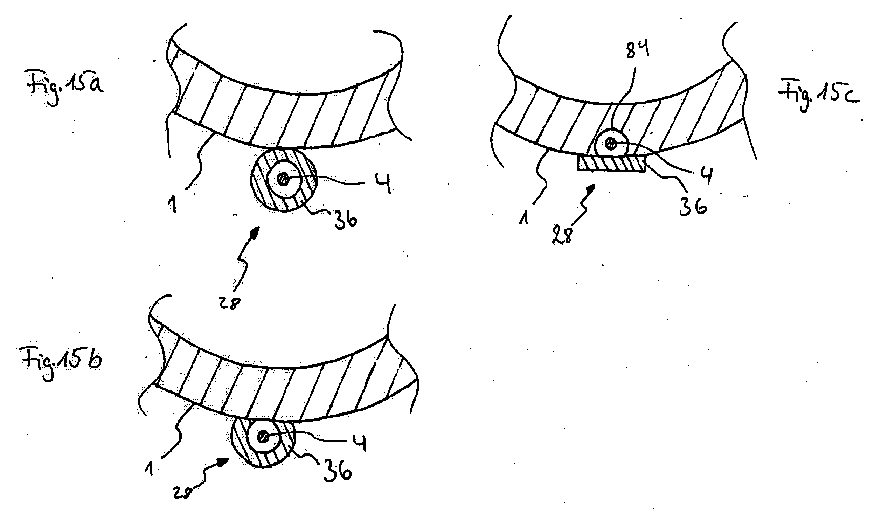

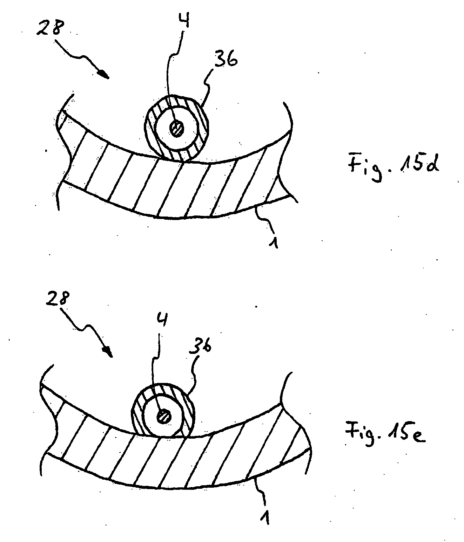

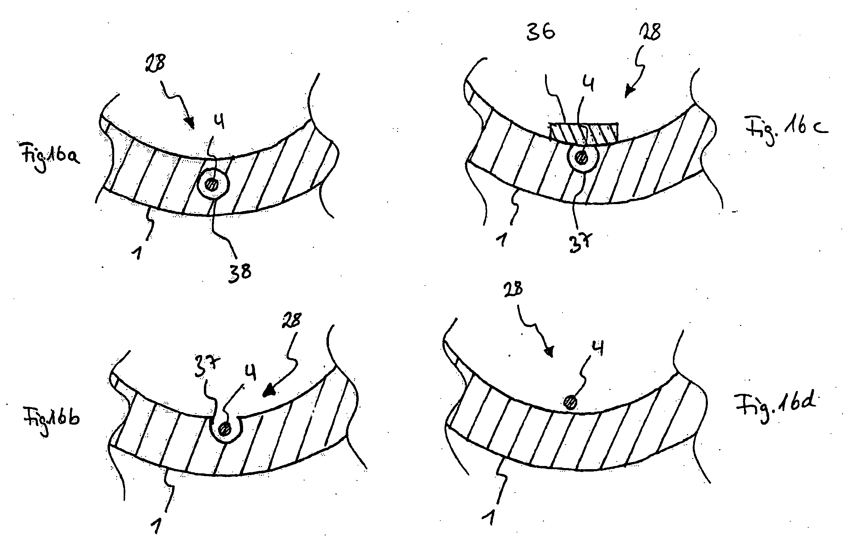

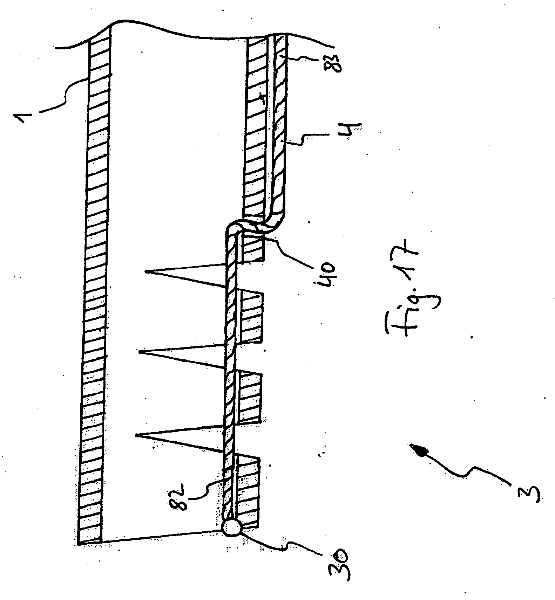

In einer vorteilhaften Ausgestaltung der zweiten Zugelementführung 28 weist die zweite Zugelementführung 28 ein Führungselement 36 auf, das einen röhrenförmigen Querschnitt hat, auf der Außenseite des inneren Rohrelements 1 angebracht ist und im Inneren des röhrenförmigen Querschnitts das Zugelement 4 führt (siehe Fig. 15a). In einer weiteren vorteilhaften Ausgestaltung der zweiten Zugelementführung 28 weist die zweite Zugelementführung 28 ein Führungselement 36 auf, das einen röhrenförmigen Querschnitt hat, auf der Innenseite des inneren Rohrelements 1 angebracht ist und im Inneren des röhrenförmigen Querschnitts das Zugelement 4 führt (siehe Fig. 15d). In einer weiteren vorteilhaften Ausgestaltung der zweiten Zugelementführung 28 weist die zweite Zugelementführung 28 ein Führungselement 36 auf, das einen U-förmigen Querschnitt hat, derart an der Außenseite des inneren Rohrelements 1 befestigt ist, dass die offene Seite des U-förmigen Querschnitts an der Außenseite des inneren Rohrelements 1 zu liegen kommt und im Inneren des U-förmigen Querschnitts das Zugelement 4 führt (siehe Fig. 15b). In einer weiteren vorteilhaften Ausgestaltung der zweiten Zugelementführung 28 weist die zweite Zugelementführung 28 ein Führungselement 36 auf, das einen U-förmigen Querschnitt hat, derart an der Innenseite des inneren Rohrelements 1 befestigt ist, dass die offene Seite des U-förmigen Querschnitts an der Innenseite des inneren Rohrelements 1 zu liegen kommt und im Inneren des U-förmigen Querschnitts das Zugelement 4 führt (siehe Fig. 15e). In einer weiteren vorteilhaften Ausgestaltung der zweiten Zugelementführung 28 weist die Außenseite des inneren Rohrelements 1 einen Nut 84 und ein Führungselement 36 auf, wobei das Zugelement 4 in der Nut 84 geführt wird und die offene Seite der Nut 84 zumindest teilweise von dem Führungselement 36 abgedeckt wird (siehe Fig. 15c). In einer weiteren vorteilhaften Ausgestaltung der zweiten Zugelementführung 28 weist die Wand des inneren Rohrelements 1 eine durchgehende, der Achse 9 des Instruments 8 parallele Bohrung 38 auf, in der das Zugelement 4 geführt wird siehe Fig. 16a . In einer weiteren vorteilhaften Ausgestaltung der zweiten Zugelementführung 28 weist die Innenseite des inneren Rohrelements 1 eine Nut 37 auf, in der das Zugelement 4 geführt wird (siehe Fig. 16b). In einer weiteren vorteilhaften Ausgestaltung der zweiten Zugelementführung 28 weist die Innenseite des inneren Rohrelements 1 eine Nut 37 auf, in der das Zugelement 4 geführt wird und die von einem Führungselement 36 zumindest teilweise abgedeckt wird (siehe Fig. 16c). In einer weiteren vorteilhaften Ausgestaltung der zweiten Zugelementführung 28 wird das Zugelement 4 im Inneren des inneren Rohrelements 1 geführt (siehe Fig. 16d). In einer weiteren vorteilhaften Ausgestaltung der zweiten Zugelementführung 28 wird das Zugelement 4 proximal der ersten Strukturen 3 durch eine zweite seitliche Bohrung 40 in der Wand des inneren Rohrelements 1 geführt (siehe Fig. 17).

Zur Übertragung der Kraft zur Abwinkelung der Instrumentenspitze 80 von dem proximalen Ende 6 des Instruments 8 auf das Zugelement 4 werden das innere Rohrelement 1 und das äußere Rohrelement 2 parallel zur Achse 9 des Instruments 8 gegeneinander verschoben. Um einen achsensymmetrischen Querschnitt zu erzeugen, wird das innere Rohrelement 1 in dem äußeren Rohrelement 2 geführt. Die Außenseite 43 des inneren Rohrelements 1 steht dabei vorzugsweise in direktem Kontakt mit der Innenseite 42 des äußeren Rohrelements 2 . Es kann vorteilhaft sein, die Reibung zwischen der Außenseite 43 des inneren Rohrelements 1 und der Innenseite 42 des äußeren Rohrelements 2 durch Verkleinerung der Kontaktfläche zu reduzieren. Des Weiteren kann es vorteilhaft sein, die Querschnittsform des inneren Rohrelements 1 und die Querschnittsform des äußeren Rohrelements 2 derart zu gestalten, dass eine Rotation des inneren Rohrelements 1 relativ zum äußeren Rohrelement 2 um die Achse 9 des Instruments 8 blockiert wird.

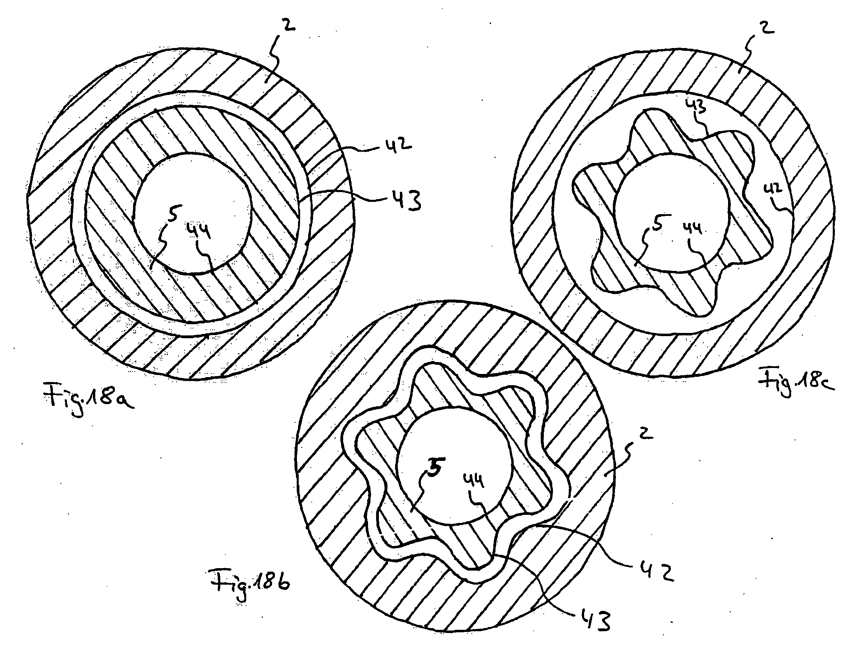

In einer vorteilhaften Ausgestaltung des inneren Rohrelements 1 und des äußeren Rohrelements 2 weist die Außenseite 43 des inneren Rohrelements 1 und die Innenseite des äußeren Rohrelements 2 einen kreisrunden Querschnitt auf (siehe Fig. 18a). In einer weiteren vorteilhaften Ausgestaltung des inneren Rohrelements 1 und des äußeren Rohrelements 2 weisen die Außenseite 43 des inneren Rohrelements 1 und die Innenseite des äußeren Rohrelements 2 Querschnittsformen auf, die eine Rotation des inneren Rohrelements 1 relativ zum äußeren Rohrelement 2 um die Achse 9 des Instruments 8 blockieren. Diese Querschnittsformen können beispielsweise ein Mehr-Eck sein, oder sie können sternenförmig sein. Wahlweise weisen diese Querschnittsformen Rundungen auf (siehe Fig. 18b).

In einer weiteren vorteilhaften Ausgestaltung des inneren Rohrelements 1 und des äußeren Rohrelements 2 weist die Innenseite 42 des äußeren Rohrelements 2 einen kreisrunden Querschnitt auf und weist die Außenseite 43 des inneren Rohrelements 1 eine Querschnittsform auf, die nicht kreisrund ist, beispielsweise ein Mehr-Eck, oder der Querschnitt ist sternenförmig. Wahlweise weist diese Querschnittsform Rundungen auf (siehe Fig. 18c).

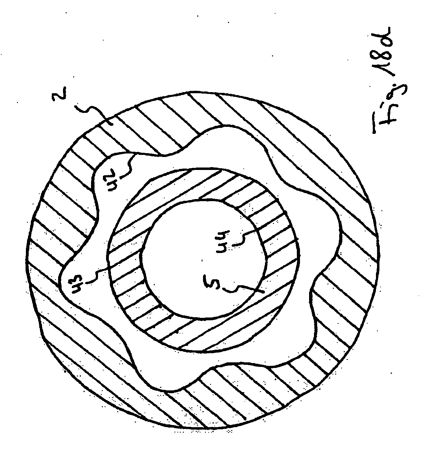

In einer weiteren vorteilhaften Ausgestaltung des inneren Rohrelements 1 und des äußeren Rohrelements 2 weist die Außenseite 43 des inneren Rohrelements 1 einen kreisrunden Querschnitt auf und weist die Innenseite 42 des äußeren Rohrelements 2 eine Querschnittsform auf, die nicht kreisrund ist, beispielsweise ein Mehr-Eck oder sternenförmig. Wahlweise weist diese Querschnittsformen Rundungen auf (siehe Fig. 18d).

Das Instrument 8 dient zur Führung/Einführung einer zweiten medizinischen Vorrichtung 45 , die einen chirurgischen Effektor 48 aufweist. Der chirurgische Effektor 48 kann beispielsweise eine Fasszange, eine Biopsiezange, ein Nadelhalter, ein Nähvorrichtung, ein Klammer-Applikator, eine Schere, eine Schlinge, ein Beutel, ein Clip-Applikator, eine Injektionsnadel, eine Klinge, eine Siebvorrichtung, eine Leuchteinheit, eine Hochfrequenzstrom-Schneidevorrichtung, eine Laser-Schneidevorrichtung, ein Ballonapplikator, ein Stentapplikator, eine Wasserstrahl-Dissektionsvorrichtung, eine Hochfrequenzstrom-Koagulationsvorrichtung, eine Argon-Plasma-Koagulationsvorrichtung, eine Laser-Koagulationsvorrichtung, eine Ultraschall-Koagulationsvorrichtung, eine Kameraeinheit, eine Hakenvorrichtung, eine Sprühvorrichtung, eine Spülvorrichtung, eine Saugvorrichtung, eine Elektrode, oder eine sensorische Sonde sein.

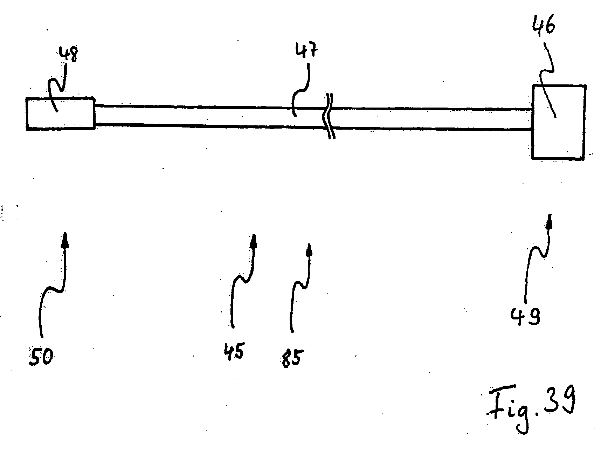

Ein Beispiel für eine zweite Vorrichtung 45 ist ein chirurgisches Instrument 85 , das einen chirurgischen Effektor 48 , einen Schaft 47 und ein fünftes Bedienelement 46 aufweist. Das fünfte Bedienelement 46 ist dabei über den flexiblen Schaft 47 mit dem chirurgischen Effektor 48 verbunden (siehe Fig. 39). Durch Betätigung des fünften Bedienelements 46 kann die bestimmungsgemäße Funktion des chirurgischen Effektors 48 eingestellt werden. Das fünfte Bedienelement 46 befindet sich am proximalen Ende 49 des chirurgischen Instruments 85 und der chirurgische Effektor 48 befindet sich am distalen Ende 50 des chirurgischen Instruments 85 . Das chirurgische Instrument 85 wird vorzugsweise derart in dem Instrument 8 positioniert, dass der Schaft 47 des chirurgischen Instruments 85 in dem inneren Rohrelement 1 geführt wird und das distale Ende 50 des chirurgischen Instruments 85 wahlweise aus der Instrumentenspitze 80 herausgeführt werden kann (siehe Fig. 19).

Das chirurgische Instrument 85 ist parallel zur Achse 9 des Instruments 8 verschiebbar. Die Außenseite 51 des chirurgischen Instruments 85 und die Innenseite 44 des inneren Rohrelements 1 stehen dabei vorzugsweise in direktem Kontakt miteinander. Es kann vorteilhaft sein, die Reibung zwischen der Außenseite 51 des chirurgischen Instruments 85 und der Innenseite 44 des inneren Rohrelements 1 durch Verkleinerung der Kontaktfläche zu reduzieren. Des Weiteren kann es vorteilhaft sein, die Querschnittsform der Außenseite 51 des chirurgischen Instruments 85 und die Querschnittsform der Innenseite 44 des inneren Rohrelements 1 derart zu gestalten, dass eine Rotation des chirurgischen Instruments 85 relativ zum inneren Rohrelement 1 um die Achse 9 des Instruments 8 blockiert wird.

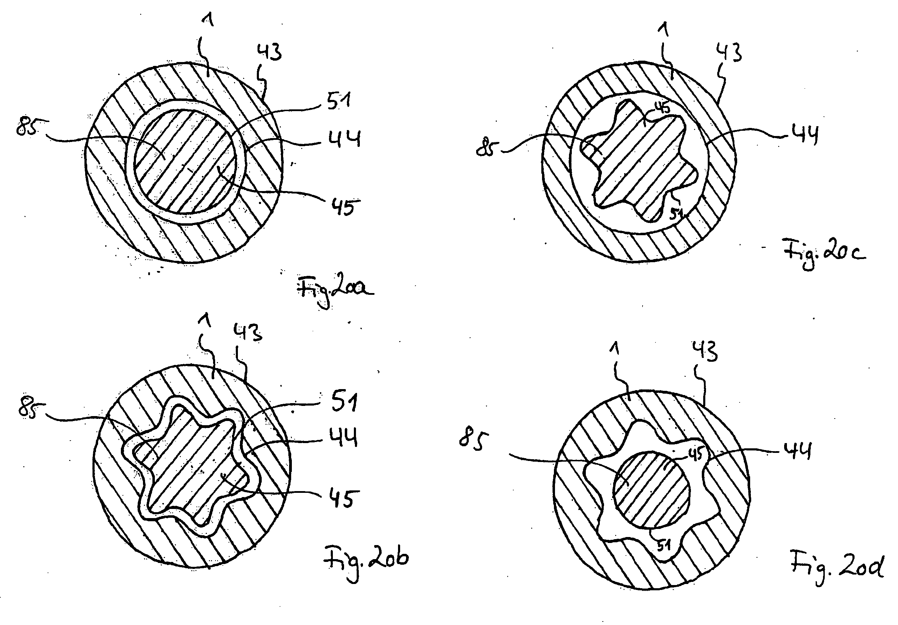

In einer vorteilhaften Ausgestaltung des chirurgischen Instruments 85 und des inneren Rohrelements 1 weist die Außenseite 51 des chirurgischen Instruments 85 und die Innenseite 44 des inneren Rohrelements 1 einen kreisrunden Querschnitt auf (siehe Fig. 20a). In einer weiteren vorteilhaften Ausgestaltung des chirurgischen Instruments 85 und des inneren Rohrelements 1 weisen die Außenseite 51 des chirurgischen Instruments 85 und die Innenseite 44 des inneren Rohrelements 1 Querschnittsformen auf, die eine Rotation des chirurgischen Instruments 85 relativ zum inneren Rohrelement 1 um die Achse 9 des Instruments 8 blockieren. Diese Querschnittsformen können beispielsweise ein Mehr-Eck sein oder sie können sternenförmig sein. Wahlweise weisen diese Querschnittsformen Rundungen auf siehe Fig. 20b . In einer weiteren vorteilhaften Ausgestaltung des chirurgischen Instruments 85 und des inneren Rohrelements 1 weist die Innenseite 44 des inneren Rohrelements 1 einen kreisrunden Querschnitt auf und weist die Außenseite 51 des chirurgischen Instruments 85 eine Querschnittsform auf, die nicht kreisrund ist, beispielsweise ein Mehr-Eck oder sternenförmig. Wahlweise weist diese Querschnittsform Rundungen auf (siehe Fig. 20c). In einer weiteren vorteilhaften Ausgestaltung des chirurgischen Instruments 85 und des inneren Rohrelements 1 weist die Außenseite 51 des chirurgischen Instruments 85 einen kreisrunden Querschnitt auf, und weist die Innenseite 44 des inneren Rohrelements leine Querschnittsform auf, die nicht kreisrund ist, beispielsweise ein Mehr-Eck oder sternenförmig. Wahlweise weist diese Querschnittsform Rundungen auf (siehe Fig. 20d).

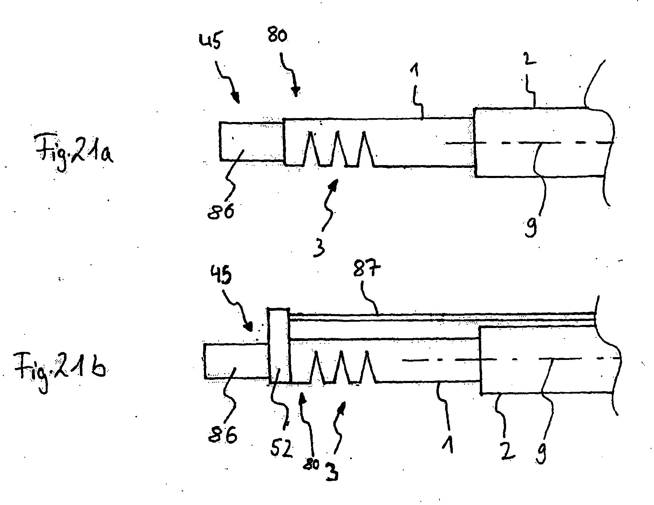

Ein weiteres Beispiel für eine zweite Vorrichtung 45 ist ein chirurgischer Effektor 86 , der an der Instrumentenspitze 80 befestigt ist. Der chirurgische Effektor 86 kann beispielsweise eine Fasszange, eine Biopsiezange, ein Nadelhalter, ein Nähvorrichtung, ein Klammer-Applikator, eine Schere, eine Schlinge, ein Beutel, ein Clip-Applikator, eine Injektionsnadel, eine Klinge, eine Siebvorrichtung, eine Leuchteinheit, eine Hochfrequenzstrom-Schneidevorrichtung, eine Laser-Schneidevorrichtung, ein Ballonapplikator, ein Stentapplikator, eine Wasserstrahl-Dissektionsvorrichtung, eine Hochfrequenzstrom-Koagulationsvorrichtung, eine Argon-Plasma-Koagulationsvorrichtung, eine Laser-Koagulationsvorrichtung, eine Ultraschall-Koagulationsvorrichtung, eine Kameraeinheit, eine Hakenvorrichtung, eine Sprühvorrichtung, eine Spülvorrichtung, eine Saugvorrichtung, eine Elektrode, oder eine sensorische Sonde sein. Bei einer Abwinkelung der Instrumentenspitze 80 folgt der chirurgische Effektor 86 der Abwinkelung. Auf diese Weise kann der chirurgische Effektor 86 ausgerichtet werden (siehe Fig. 21a).

In einer vorteilhaften Ausgestaltung des chirurgischen Effektors 86 ist dieser über eine vierte Vorrichtung 52 mit der Instrumentenspitze 80 verbunden. Diese vierte Vorrichtung 52 ermöglicht eine Rotation des chirurgischen Effektors 86 um die Achse 9 des Instruments 8 .

Eine beispielhafte Ausführung der vierten Vorrichtung 52 weist mechanische Getriebeelemente 39 auf, die derart gestaltet sind, dass die Rotation des chirurgischen Effektors 86 über ein Kraftübertragungselement 87 beispielsweise in Form einer biegeflexiblen Drehwelle eingestellt werden kann. Das Kraftübertragungselement 87 führt dabei von der vierten Vorrichtung 52 bis zum proximalen Ende 6 des Instruments 8 . Die vierte Vorrichtung 52 ist derart gestaltet, dass durch Kraft-oder Drehmoment-Einleitung in das Kraftübertragungselement 87 die Rotation des chirurgischen Effektors 86 eingestellt werden kann (siehe Fig. 21b).

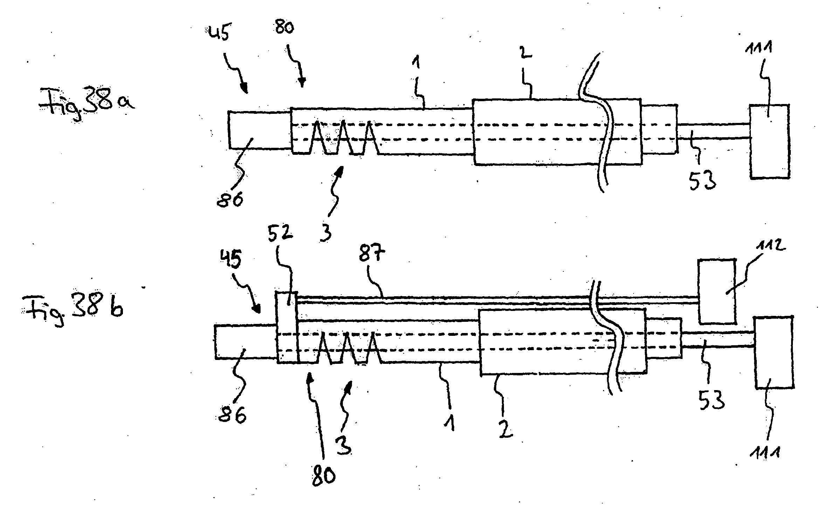

In einer vorteilhaften Ausgestaltung der vierten Vorrichtung 52 weist das Kraftübertragungselement 87 ein sechstes Bedienelement 112 auf, das derart mit dem Kraftübertragungselement 87 verbunden ist, dass bei Betätigung des sechsten Bedienelements 112 die Rotation des chirurgischen Effektors 86 eingestellt werden kann (siehe Fig. 38b).

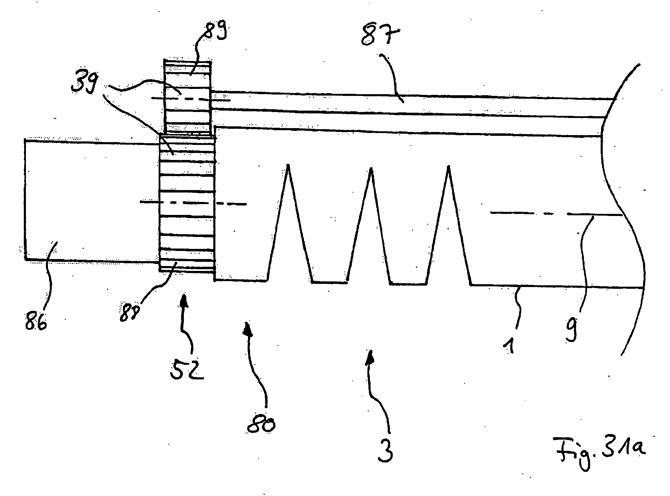

Eine vorteilhafte Ausgestaltung der mechanischen Getriebeelemente 39 und des Kraftübertragungselements 87 weist zwei Stirnzahnräder auf, von denen ein erstes Stirnzahnrad 88 um die Achse 9 des Instruments 8 drehbar gelagert ist und mit dem chirurgischen Effektor 86 drehfest verbunden ist, und von denen ein zweites Stirnzahnrad 89 derart drehbar gelagert ist, dass es mit dem ersten Stirnzahnrad 88 eine Getriebeeinheit bildet, und mit dem Kraftübertragungselement 87 drehfest verbunden ist. Das Kraftübertragungselement 87 ist dabei in Form einer Welle derart gestaltet, dass es ein am proximalen Ende 6 des Instruments 8 in das Kraftübertragungselement 87 eingeleitetes Drehmoment auf das zweite Stirnzahnrad 89 überträgt. Vorzugsweise weist das erste Stirnzahnrad 88 einen größeren Durchmesser als das zweite Stirnzahnrad 89 auf, um ein Übersetzungsverhältnis zwischen der Rotationsgeschwindigkeit des Kraftübertragungselements 87 und des chirurgischen Effektors 86 von größer eins einzustellen (siehe Fig. 31a).

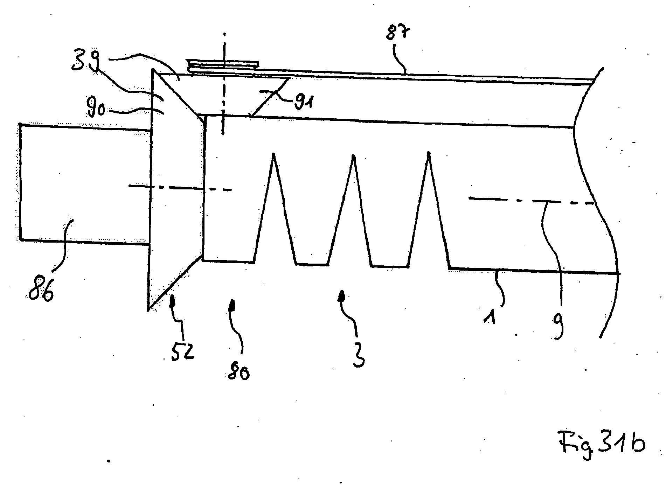

Eine weitere vorteilhafte Ausgestaltung der mechanischen Getriebeelemente 39 und des Kraftübertragungselements 87 weist zwei Kegelzahnräder auf, von denen ein erstes Kegelzahnrad 90 um die Achse 9 des Instruments 8 drehbar gelagert ist und mit dem chirurgischen Effektor 86 drehfest verbunden ist, und von denen ein zweites Kegelzahnrad 91 derart drehbar gelagert ist, dass es mit dem ersten Kegelzahnrad 90 eine Getriebeeinheit bildet, und mit dem Kraftübertragungselement 87 wirkverbunden ist. Das Kraftübertragungselement 87 ist dabei in Form eines Riemenantriebs derart gestaltet, dass es eine am proximalen Ende 6 des Instruments 8 in das Kraftübertragungselement 87 eingeleitete Zugkraft auf das zweite Kegelzahnrad 91 derart überträgt, dass das zweite Kegelzahnrad 91 in Rotation versetzt wird. Vorzugsweise ist das Kraftübertragungselement 87 als Seilzug ausgeführt, wobei die Drehachse des zweiten Kegelzahnrads 91 im Wesentlichen senkrecht zur Instrumentenachse steht. Vorzugsweise weist das erste Kegelzahnrad 90 einen größeren Durchmesser als das zweite Kegelzahnrad 91 auf, um ein Übersetzungsverhältnis zwischen der Rotationsgeschwindigkeit des zweiten Kegelzahnrads 91 und des ersten Kegelzahnrads 90 von größer eins einzustellen und damit die für ein bestimmtes Drehmoment des chirurgischen Effektors 86 erforderliche Zugkraft im Kraftübertragungselement 87 zu reduzieren (siehe Fig. 31b).

Der chirurgische Effektor 86 weist wahlweise ein erstes Steuerelement 53 und ein siebtes Bedienelement 111 auf. Das siebte Bedienelement 111 ist derart mit dem ersten Steuerelement 53 verbunden, dass bei Betätigung des siebten Bedienelements 111 die bestimmungsgemäße Funktion des chirurgischen Effektors 86 , vorzugsweise vom proximalen Ende 6 des Instruments 8 aus, eingestellt werden kann (siehe Fig. 38a).

In einer vorteilhaften Ausgestaltung des ersten Steuerelements 53 kann das erste Steuerelement wahlweise derart eingestellt werden, dass die Einstellung der bestimmungsgemäßen Funktion des chirurgischen Effektors 86 blockiert ist. Dadurch kann eine bevorzugte Einstellung der bestimmungsgemäßen Funktion des chirurgischen Effektors 86 ohne Betätigung des siebten Bedienelements 111 beibehalten werden. In einer weiteren vorteilhaften Ausgestaltung des ersten Steuerelements 53 ist das erste Steuerelement 53 ein Metalldraht, über den wahlweise eine lineare Kraft oder ein Drehmoment vom siebten Bedienelement 111 zum chirurgischen Effektor 86 übertragen werden kann. In einer weiteren vorteilhaften Ausgestaltung des ersten Steuerelements 53 ist das erste Steuerelement 53 ein Faden, über den eine Zugkraft vom siebten Bedienelement 111 zum chirurgischen Effektor 86 übertragen werden kann. In einer weiteren vorteilhaften Ausgestaltung des ersten Steuerelements 53 besteht das erste Steuerelement 53 aus mindestens einem stromleitenden Kabel, über das elektrische Signale vom siebten Bedienelement 111 zum chirurgischen Effektor 86 übertragen werden können. Dies können beispielsweise analoge Messsignale wie Spannungen oder Ströme, digitale Daten oder Hochfrequenzstrom zum Betreiben eines Hochfrequenz-Effektors sein.

In einer vorteilhaften Ausgestaltung der vierten Vorrichtung 52 kann die Einstellung der Rotation des chirurgischen Effektors 86 wahlweise blockiert werden. Dadurch kann eine bevorzugte Ausrichtung der vierten Vorrichtung 52 ohne Betätigung des sechsten Bedienelements 112 beibehalten werden.

### Steuervorrichtung

Die Steuervorrichtung 55 dient zur manuellen Steuerung des Instruments 8 in verschiedenen Ausführungen. Die Steuervorrichtung 55 dient im Einzelnen zur manuellen Einstellung der Abwinkelung der Instrumentenspitze 80 , wahlweise zur manuellen Einstellung des Vorschubs eines chirurgischen Instruments 85 parallel zur Achse 9 des Instruments 8 , wahlweise zur manuellen Einstellung der Rotation eines chirurgischen Instruments 85 um die Achse 9 des Instruments 8 , wahlweise zur manuellen Einstellung der bestimmungsgemäßen Funktion eines chirurgischen Effektors 48 , wahlweise zur manuellen Einstellung der Rotation eines chirurgischen Effektors 86 , wahlweise zur manuellen Einstellung der bestimmungsgemäßen Funktion eines chirurgischen Effektors 86 , wahlweise zur manuellen Einstellung des Vorschubs des Instruments 8 parallel zur Achse 9 des Instruments 8 und wahlweise zur manuellen Einstellung der Rotation des Instruments 8 um die Achse 9 des Instruments 8 .

Die Steuervorrichtung 55 weist eine siebte Vorrichtung 57 zur Einleitung einer Schubkraft in das innere und äußere Rohrelement des Instruments und ein erstes Bedienelement 59 zur manuellen/elektromotorischen Aufbringung einer Schubkraft auf. Die siebte Vorrichtung 57 hat zwei Verbindungselemente vorzugsweise in Form von Klemmschellen, Steckringen etc., wobei das erste Verbindungselement 54 eine Verbindung zwischen dem ersten Bedienelement 59 und dem äußeren Rohrelement 2 des Instruments 9 herstellt, und das zweite Verbindungselement 92 eine Verbindung zwischen dem ersten Bedienelement 59 und dem inneren Rohrelement 1 des Instruments 8herstellt. Das erste Verbindungselement 54 und das zweite Verbindungselement 92 sind derart gestaltet, dass sie sich relativ zueinander parallel zur Achse 9 des Instruments 8 verschieben lassen. Das erste Verbindungselement 54 und das zweite Verbindungselement 92 sowie das ersten Bedienelement 59 sind dabei derart mit miteinander gekoppelt, dass durch Betätigung des ersten Bedienelements 59 eine Verschiebung des ersten Verbindungselements 54 relativ zum zweiten Verbindungselement 92 eingestellt werden kann. Das erste Verbindungselement 54 und das zweite Verbindungselement 92 sind dabei derart mit dem Instrument 8 verbunden, dass bei einem Verschieben des ersten Verbindungselements 54 relativ zum zweiten Verbindungselement 92 auch das innere Rohrelement 1 relativ zum äußeren Rohrelement 2 verschoben wird und dadurch eine Abwinkelung der Instrumentenspitze 80 eingestellt werden kann.

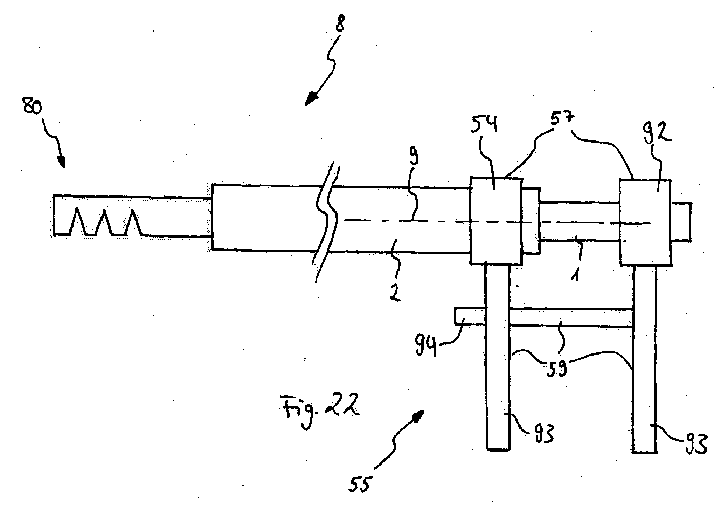

In einer vorteilhaften Ausgestaltung des ersten Bedienelements 59 besteht das erste Bedienelement 59 aus zwei stab-oder abzugsförmigen Griffen 93 , von denen einer mit dem ersten Verbindungselement 54 fest verbunden ist und der andere mit dem zweiten Verbindungselement 92 fest verbunden ist. Durch Verschieben der Griffe 93 relativ zueinander kann das erste Verbindungselement 54 relativ zum zweiten Verbindungselement 92 verschoben werden. Dadurch kann eine Einstellung der Abwinkelung der Instrumentenspitze 80 vorgenommen werden. Wahlweise kann die Verschiebung der Griffe 93 von einem Führungselement 94 geführt werden, siehe Fig. 22 . Dieses besteht beispielsweise aus einer Führungsstange, die sich längs der Bewegungsrichtung der beiden Griffe erstreckt, und die an einem Griff befestigt sowie in/an dem anderen Griff gleitend gelagert ist.

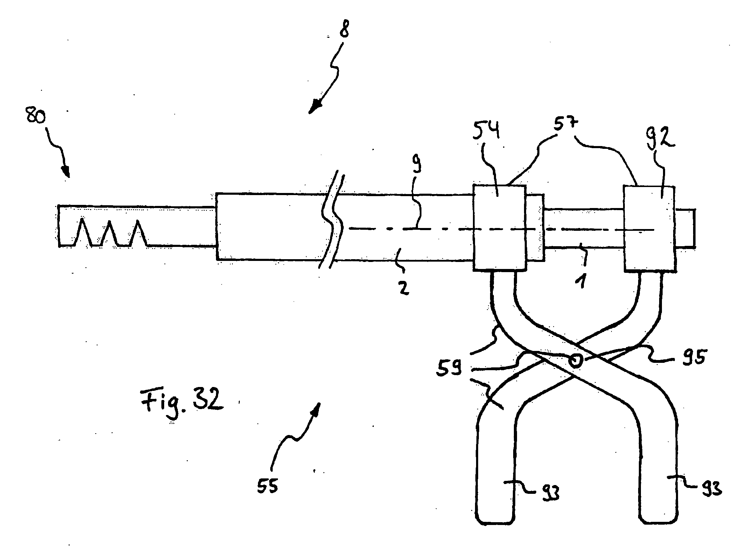

In einer weiteren vorteilhaften Ausgestaltung des ersten Bedienelements 59 weist das erste Bedienelement 59 zwei stabförmige Griffe 93 auf, von denen einer mit dem ersten Verbindungselement 54 fest verbunden ist und der andere mit dem zweiten Verbindungselement 92 fest verbunden ist. Die beiden Griffe 93 sind zusätzlich untereinander über einen Bolzen oder Zapfen 95 drehbar nach dem Scherenprinzip miteinander verbunden. Durch Drehen der beiden Griffe 93 relativ zueinander um den Zapfen 95 kann das erste Verbindungselement 54 relativ zum zweiten Verbindungselement 92 verschoben werden. Dadurch kann eine Einstellung der Abwinkelung der Instrumentenspitze 80 vorgenommen werden (siehe Fig. 32).

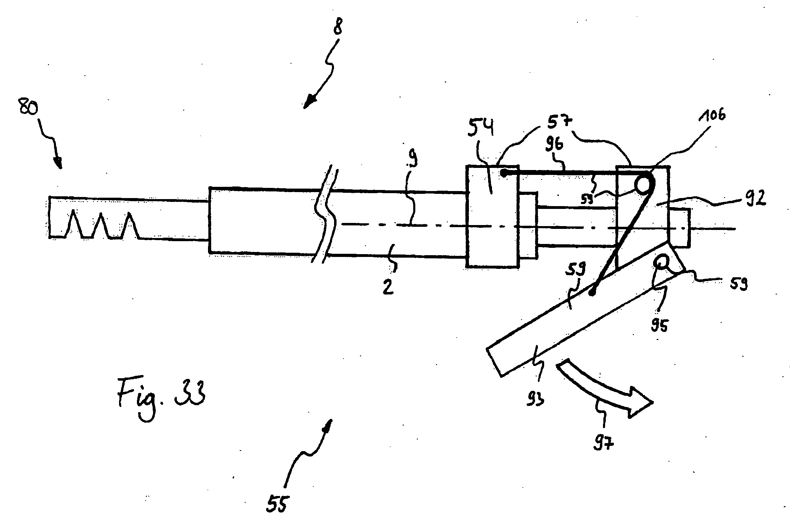

In einer weiteren vorteilhaften Ausgestaltung des ersten Bedienelements 59 weist das erste Bedienelement 59 einen Griff 93 in Form eines Abzugs auf, der über einen Schwenkzapfen 95 einseitig mit dem einen Verbindungselement 57 schwenkbar verbunden ist. Des Weiteren ist der Griff 93 über ein zweites Kraftübertragungselement 96 in Form eines Seilzugs mit dem anderen Verbindungselement wirkverbunden, derart, dass bei Drehung des Griffes 3 um den Schwenkzapfen 95 in eine Schwenkrichtung 97 das erste Verbindungselement 54 an das zweite Verbindungselement 92 herangezogen wird. Hierfür ist das Zugseil 96 in einem Mittenabschnitt des Abzugs oder Hebels 59 fixiert und über eine Umlenkung 106 geführt, welche an dem den Schwenkzapfen 95 tragenden Verbindungselement angeordnet ist (siehe Fig. 33). In einer weiteren vorteilhaften Ausgestaltung des ersten Bedienelements 59 weist das erste Bedienelement 59 Getriebeelemente, beispielsweise ein Zahnrad, eine Zahnstange oder einen Zahnriemen auf. In einer weiteren vorteilhaften Ausgestaltung des ersten Bedienelements 59 weist das erste Bedienelement 59 mindestens einen Hebelmechanismus auf.

In einer beispielhaften Ausführung der Umlenkung 106 besteht die Umlenkung 106 aus einer drehbar an dem einen Verbindungselement gelagerten Umlenkrolle, in der das Kraftübertragungselement 96 geführt ist. In einer weiteren beispielhaften Ausführung der Umlenkung 106 besteht die Umlenkung 106 aus einer statischen mechanischen Barriere, die das Kraftübertragungselement 96 verformt. In einer weiteren beispielhaften Ausführung der Umlenkung 106 besteht die Umlenkung 106 aus einem Rohrelement, in dem das Kraftübertragungselement 96 geführt wird.

In einer beispielhaften Ausführung des Kraftübertragungselements 96 ist das Kraftübertragungselement 96 ein Zugfaden oder ein Zugseil. In einer weiteren beispielhaften Ausführung des Kraftübertragungselements 96 ist das Kraftübertragungselement 96 ein Draht.

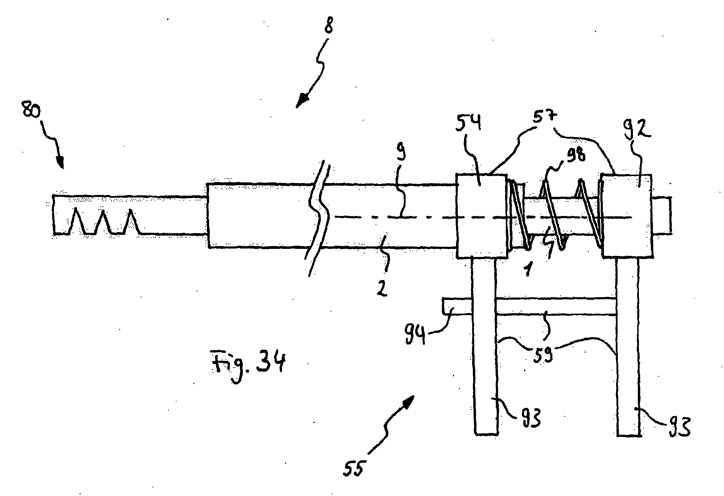

In einer vorteilhaften Ausgestaltung der siebten Vorrichtung 57 weist die siebte Vorrichtung ein Federelement 98 auf, das zwischen den beiden Verbindungselementen angeordnet ist und bei Annäherung des ersten Verbindungselements 54 an das zweite Verbindungselement 92 gestaucht wird (siehe Fig. 34).

Die Steuervorrichtung 55 weist wahlweise eine achte Vorschubvorrichtung 62 und ein zweites Bedienelement 63 auf. Die achte Vorschubvorrichtung 62 hat ein drittes, vorzugsweise Klemmring-artiges Verbindungselement 99 , das mit dem chirurgischen Instrument 85 verbunden ist. Die Verbindung zwischen dem dritten Verbindungselement 99 und dem chirurgischen Instrument 85 ist derart gestaltet, dass eine Verschiebung des dritten Verbindungselements 99 längs der Achse 9 des Instruments 8 eine Verschiebung des chirurgischen Instruments 85 längs der Achse 9 des Instruments 8 bewirkt. Das zweite Bedienelement 63 dient dabei zur Längsverschiebung des dritten Verbindungselements 99 und damit zur Längsverschiebung des chirurgischen Instruments 8 .

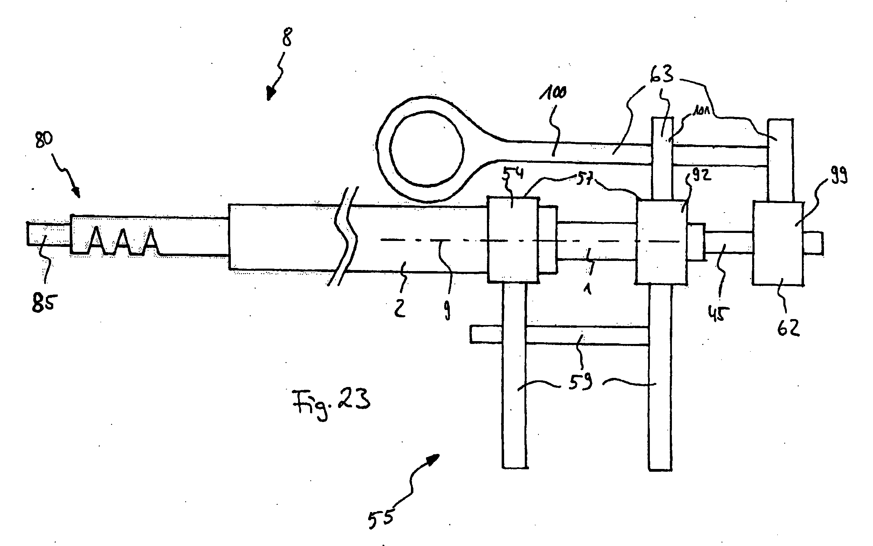

In einer vorteilhaften Ausführung des zweiten Bedienelements 63 weist das zweite Bedienelement 63 eine Zugstange oder Seilzug jeweils mit Griff 100 auf, der an dem dritten Verbindungselement 99 über einen Bolzen angelenkt ist, derart, dass durch Verschieben der Zugstange 100 das dritte Verbindungselement 99 parallel zur Achse 9 des Instruments 8 verschoben werden kann. Dadurch kann das chirurgische Instrument 85 relativ zum Instrument 8 verschoben werden. Wahlweise kann die Zugstange mit Griff 100 von einem Führungselement 101 beispielsweise in Form einer Öse oder Hülse geführt werden, die vorzugsweise am Verbindungselement des inneren Rohrelements des Instruments befestigt ist (siehe Fig. 23).

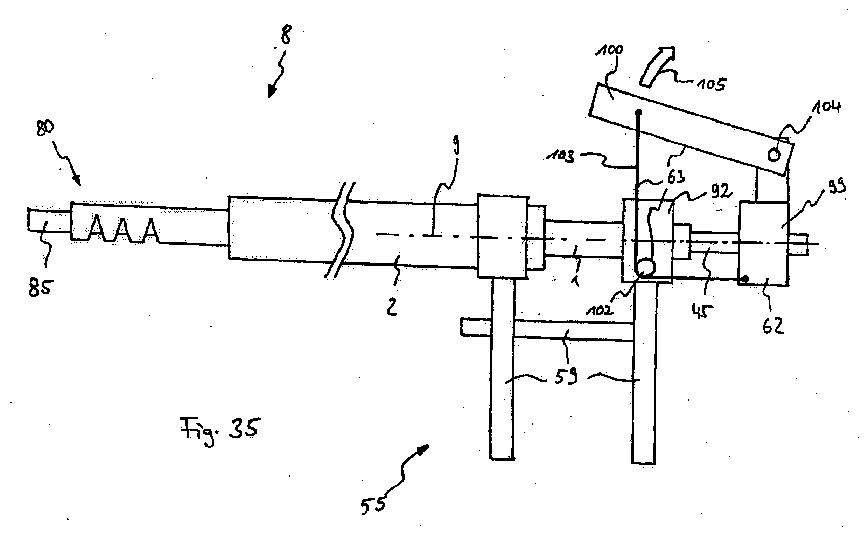

In einer weiteren vorteilhaften Ausführung des zweiten Bedienelements 63 weist das zweite Bedienelement 63 einen hebelförmigen Griff 100 auf, der in einer Drehvorrichtung 104 schwenkbar mit dem dritten Verbindungselement 99 verbunden ist. An einem Mittenabschnitt des Griffes 100 , ist ein Kraftübertragungselement 103 vorzugsweise in Form eines Zugseils befestigt. Das Kraftübertragungselement 103 ist des Weiteren mit dem dritten Verbindungselement 99 derart verbunden, dass bei Verschwenken des Griffes 100 in die Vorzugsrichtung 105 das dritte Verbindungselement 99 relativ zum Verbindungselement des inneren Rohrelements parallel zur Achse 9 des Instruments 8 verschoben werden kann. Das Kraftübertragungselement 103 kann wahlweise über ein oder mehrere Umlenkungen 102 umgelenkt werden, die am Verbindungselement des inneren Rohrelements angeordnet sind (siehe Fig. 35).

In einer weiteren vorteilhaften Ausgestaltung des zweiten Bedienelements 63 weist das zweite Bedienelement 63 Getriebeelemente, beispielsweise ein Zahnrad, eine Zahnstange oder einen Zahnriemen auf. In einer weiteren vorteilhaften Ausgestaltung des zweiten Bedienelements 63 weist das zweite Bedienelement 63 mindestens einen Hebelmechanismus auf.

In einer beispielhaften Ausführung der Umlenkung 102 besteht die Umlenkung 102 aus einer drehbar am Verbindungselement des inneren Rohrelements gelagerten Umlenkrolle, in der das zugseilförmige Kraftübertragungselement 103 geführt ist. In einer weiteren beispielhaften Ausführung der Umlenkung 102 besteht die Umlenkung 102 aus einer statischen mechanischen Barriere, die das Kraftübertragungselement 103 verformt. In einer weiteren beispielhaften Ausführung der Umlenkung 102 besteht die Umlenkung 102 aus einem Rohrelement, in dem das Kraftübertragungselement 103 geführt wird.

In einer beispielhaften Ausführung des Kraftübertragungselements 103 ist das Kraftübertragungselement 103 ein Zugfaden oder ein Zugseil. In einer weiteren beispielhaften Ausführung des Kraftübertragungselements 103 ist das Kraftübertragungselement 103 ein Draht.

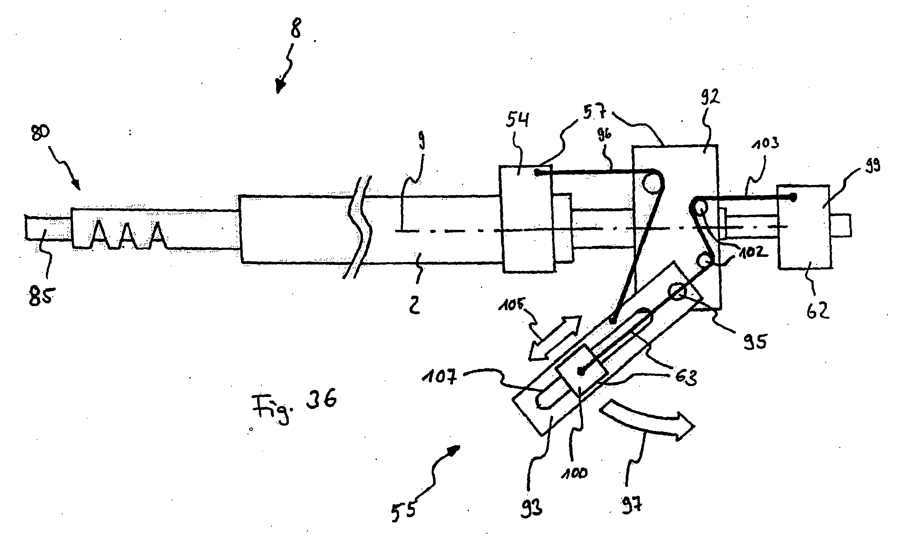

In einer weiteren vorteilhaften Ausführung des ersten Bedienelements 59 und des zweiten Bedienelements 63 weist das erste Bedienelement 59 einen hebelförmigen Griff 93 auf, der an dem zweiten Verbindungselement 92 des inneren Rohrelements des Instruments anscharniert ist. Des Weiteren ist der Griff 93 über das zweite zugseilartige Kraftübertragungselement 96 sowie der am zweiten Verbindungselement angeordneten Umlenkung mit dem ersten Verbindungselement 54 des äußeren Rohrelements des Instruments derart wirkverbunden, dass bei Verschwenken des Griffes 3 um das Scharnier 95 in die bevorzugte Drehrichtung 97 das erste Verbindungselement 54 an das zweite Verbindungselement 92 herangezogen wird. Das Kraftübertragungselement 96 kann, wie bereits angedeutet wurde, wahlweise über eine oder mehrere Umlenkvorrichtungen 106 umgelenkt werden. In das erste Bedienelement 59 ist gemäß dieser Ausführungsform das zweite Bedienelement integriert. Hierfür weist das erste Bedienelement des Weiteren eine Führung längs des hebelförmigen ersten Bedienelements auf, die derart gestaltet ist, dass sie den Griff 100 des zweiten Bedienelements 63 aufnehmen kann. Die Führung 107 , vorliegend in Form eines Längsschlitzes erlaubt eine Verschiebung des Griffes 100 des zweiten Bedienelements 63 , vorzugsweise entlang des Griffes 93 des ersten Bedienelements 59 . An einem Punkt des Griffes 100 des zweiten Bedienelements ist ein seilzugartiges Kraftübertragungselement 103 angeschlossen. Das Kraftübertragungselement 103 ist des Weiteren mit dem dritten Verbindungselement 99 des chirurgischen Instruments derart verbunden, dass bei Verschieben des Griffes 100 des zweiten Bedienelements 63 in die Vorzugsrichtung 105 das dritte Verbindungselement 99 parallel zur Achse 9 des Instruments 8 relativ zum zweiten Verbindungselement 92 verschoben werden kann. Das Kraftübertragungselement 103 wird hierfür über ein oder mehrere Umlenkungen 102 umgelenkt, welche an dem zweiten Verbindungselement 92 angeordnet sind (siehe Fig. 36).

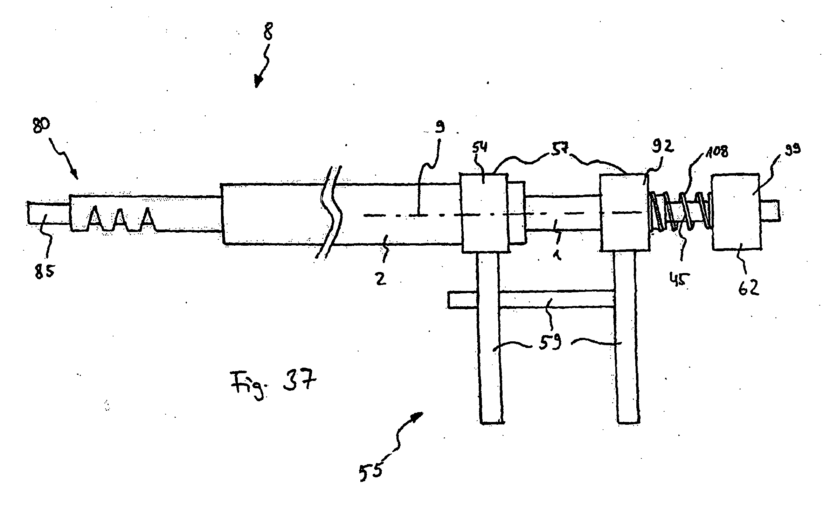

In einer vorteilhaften Ausgestaltung der achten Vorrichtung 62 weist die achte Vorrichtung 62 ein Federelement 108 auf, das zwischen dem zweiten und dritten Verbindungselement angeordnet ist und durch das bei Verschieben des dritten Verbindungselements 99 bezüglich des zweiten Verbindungselements 92 eine Rückstellkraft auf das dritte Verbindungselement 99 ausgeübt werden kann (siehe Fig. 37).

Die Steuervorrichtung 55 weist wahlweise eine neunte Vorrichtung 65 und ein drittes Bedienelement 66 zur Betätigung des chirurgischen Instruments auf. Die neunte Vorrichtung 65 hat hierfür ein viertes Verbindungselement vorzugsweise in Form eines Klemmrings 109 , das mit dem chirurgischen Instrument 85 verbunden ist. Die mechanische Verbindung zwischen dem vierten Verbindungselement 109 und dem chirurgischen Instrument 85 ist dabei derart gestaltet, dass eine Rotation des vierten Verbindungselements 109 um die Achse 9 des Instruments 8 eine Rotation des chirurgischen Instruments 85 um die Achse 9 des Instruments 8 bewirkt. Das dritte Bedienelement 66 ist des Weiteren derart mit dem vierten Verbindungselement 109 wirkverbunden, dass eine Betätigung des dritten Bedienelements 66 eine Rotation des vierten Verbindungselements 109 um die Achse 9 des Instruments 8 bewirken kann.

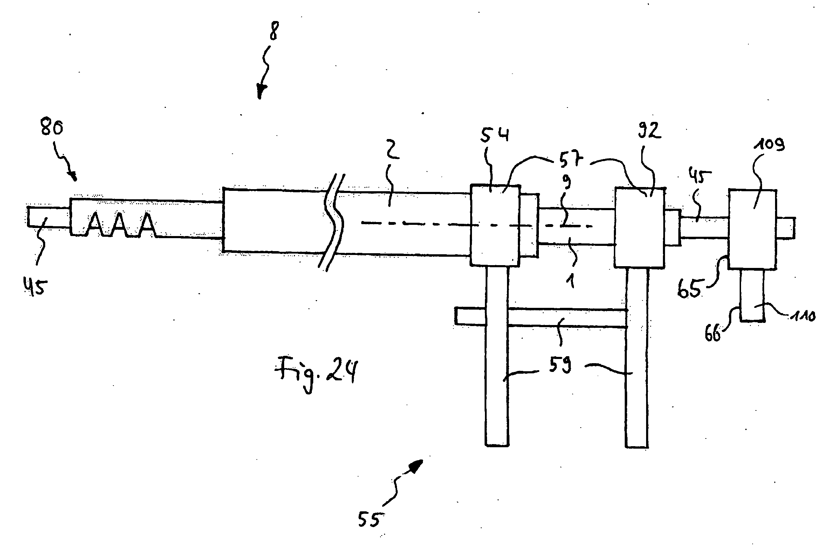

In einer vorteilhaften Ausgestaltung des dritten Bedienelements 66 weist das dritte Bedienelement 66 einen stabförmigen Griff 110 auf, der mit dem vierten Verbindungselement 99 derart wirkverbunden ist, dass durch Rotieren des Griffs 110 das vierte Verbindungselement 109 um die Achse 9 des Instruments 8 gedreht werden kann. Dadurch kann das chirurgische Instrument 85 um die Achse 9 des Instruments 8 rotiert werden. Die Achse des Griffs 110 muss dabei nicht zwingend mit der Achse 9 des Instruments 8 übereinstimmen (siehe Fig. 24) sondern kann bei Zwischenfügen eines Umlenkgetriebes auch einem Winkel zur Längsachse des Instruments 8 ausgerichtet sein. In einer weiteren vorteilhaften Ausgestaltung des dritten Bedienelements 66 weist das dritte Bedienelement 66 hierfür Getriebeelemente, beispielsweise ein Zahnrad, eine Zahnstange oder einen Zahnriemen auf. In einer weiteren vorteilhaften Ausgestaltung des dritten Bedienelements 66 weist das dritte Bedienelement 66 mindestens einen Hebelmechanismus auf. Es kann dabei vorteilhaft sein, die achte Vorrichtung 62 und die neunte Vorrichtung 65 derart miteinander zu kombinieren, dass die Steuerung der Rotation des chirurgischen Instruments 85 um die Achse 9 des Instruments 8 und der Verschiebung des chirurgischen Instruments 85 parallel zur Achse 9 des Instruments 8 über ein einziges Verbindungselement gesteuert wird, das derart mit dem chirurgischen Instrument 85 verbunden werden kann, dass sowohl eine Rotation um die Achse 9 des Instruments 8 als auch eine Verschiebung parallel zur Achse 9 des Instruments 8 auf das chirurgische Instrument 85 übertragen werden kann.

In einer weiteren vorteilhaften Ausgestaltung der siebten Vorrichtung 57 kann die siebte Vorrichtung 57 wahlweise vom ersten Bedienelement 59 entkoppelt werden. Dadurch ist eine Arbeitspunkteinstellung des ersten Bedienelements 59 möglich. In einer weiteren vorteilhaften Ausgestaltung der siebten Vorrichtung 57 kann die Einstellung des Abstandes des ersten Verbindungselements 54 zum zweiten Verbindungselement 92 wahlweise blockiert werden. Dadurch kann eine bevorzugte Abwinkelung der Instrumentenspitze 80 ohne Betätigung des ersten Bedienelements 59 beibehalten werden.

In einer weiteren vorteilhaften Ausgestaltung der achten Vorrichtung 62 kann die achte Vorrichtung 62 wahlweise von dem zweiten Bedienelement 63 entkoppelt werden. Dadurch ist eine Arbeitspunkteinstellung des zweiten Bedienelements 63 möglich. In einer weiteren vorteilhaften Ausgestaltung der achten Vorrichtung 62 kann die Einstellung der Verschiebung der achten Vorrichtung 62 parallel zur Achse 9 des Instruments 8 wahlweise blockiert werden. Dadurch kann eine bevorzugte Position des chirurgischen Instruments 85 ohne Betätigung des zweiten Bedienelements 63 beibehalten werden.

In einer vorteilhaften Ausgestaltung der neunten Vorrichtung 65 kann die neunte Vorrichtung 65 wahlweise von dem dritten Bedienelement 66 entkoppelt werden. Dadurch ist eine Arbeitspunkteinstellung des dritten Bedienelements 66 möglich. In einer weiteren vorteilhaften Ausgestaltung der neunten Vorrichtung 65 kann die Einstellung der Rotation der neunten Vorrichtung 65 um die Achse 9 des Instruments 8 wahlweise blockiert werden. Dadurch kann eine bevorzugte Position des chirurgischen Instruments 85 ohne Betätigung des dritten Bedienelements 66 beibehalten werden.

In einer vorteilhaften Ausgestaltung der Steuervorrichtung 55 bei Verwendung eines chirurgischen Instruments 85 als zweite Vorrichtung 45 ist das fünfte Bedienelement 46 mit der Steuervorrichtung 55 verbunden. Durch diese Ausgestaltung ist die Einstellung der bestimmungsgemäßen Funktion des chirurgischen Instruments 85 über das fünfte Bedienelement 46 zusammen mit der Bedienung des Instruments 8 über das erste Bedienelement 59, wahlweise das zweite Bedienelement 63 und wahlweise das dritte Bedienelement 66 in demselben Bezugssystem möglich. Dies ermöglicht bei entsprechender Ausgestaltung der Steuervorrichtung 55 die manuelle Bedienung des Instruments 8 und der Einstellung der bestimmungsgemäßen Funktion des chirurgischen Instruments 85 beispielsweise mit einer Hand.

In einer weiteren vorteilhaften Ausgestaltung der Steuervorrichtung 55 bei Verwendung eines chirurgischen Effektors 86 als zweite Vorrichtung 45 ist das siebte Bedienelement 111 mit der Steuervorrichtung 55 verbunden. Durch diese Ausgestaltung ist die Einstellung der bestimmungsgemäßen Funktion des chirurgischen Effektors 86 über das siebte Bedienelement 111 zusammen mit der Bedienung des Instruments 8 über zumindest das erste Bedienelement 59 in demselben Bezugssystem möglich. Dies ermöglicht bei entsprechender Ausgestaltung der Steuervorrichtung 55 die manuelle Bedienung des Instruments 8 und der Einstellung der bestimmungsgemäßen Funktion des chirurgischen Effektors 86 beispielsweise mit einer Hand.

In einer weiteren vorteilhaften Ausgestaltung der Steuervorrichtung 55 bei Verwendung eines chirurgischen Effektors 86 in Verbindung mit einer vierten Vorrichtung 52 als zweite Vorrichtung 45 ist das sechste Bedienelement 112 mit der Steuervorrichtung 55 verbunden. Durch diese Ausgestaltung ist die Einstellung der Rotation des chirurgischen Effektors 86 über das sechste Bedienelement 112 zusammen mit der Bedienung des Instruments 8 über zumindest das erste Bedienelement 59 in demselben Bezugssystem möglich. Dies ermöglicht bei entsprechender Ausgestaltung der Steuervorrichtung 55 die manuelle Bedienung des Instruments 8 und der Einstellung der Rotation des chirurgischen Effektors 86 beispielsweise mit einer Hand.

### Overtube

Der Overtube 68 dient zur Aufnahme und Einführung mindestens einer elften Vorrichtung 71 , vorzugsweise eines Instruments 8 , in den menschlichen Körper. Der Overtube 68 dient im Einzelnen zur Platzierung mindestens einer elften Vorrichtung und eines Kamerasystems oder visuellen Einrichtung in einem röhrenförmigen Hohlorgan, beispielsweise dem Verdauungstrakt. Vorzugsweise können zwei Instrumente 8 in dem Overtube 68 platziert werden. Das Kamerasystem kann wahlweise aus einem flexiblen Endoskop 113 oder aus einer in dem Overtube integrierten Kameraeinheit 79 bestehen. Der Overtube 68 weist am distalen Ende 69 distale Öffnungen 76 auf, aus denen die eingeführten Instrumente 8 und wahlweise das eingeführte flexible Endoskop 113 austreten können. Ein distales Endelement 73 des Overtube 68 stellt dabei das Bezugssystem der Instrumente 8 und des Kamerasystems dar.

Zur Einführung des Overtube 68 in ein röhrenförmiges Hohlorgan wie beispielsweise den Verdauungstrakt ist eine gute Flexibilität des Overtube 68 vorteilhaft, insbesondere bei der Passage stark gekrümmter röhrenförmiger Hohlorgane wie beispielsweise den Dickdarm. Gleichzeitig ist wiederum eine gute Kontrolle des durch das distale Endelement 73 des Overtube 68 dargestellten Bezugssystems im Sinne einer Kontrolle über Ausrichtung und Position des distalen Endelements 73 des Overtube 68 vorteilhaft. Die Verbindung einer guten Flexibilität des Overtube mit einer guten Kontrollierbarkeit des distalen Endelements 73 des Overtube 68 vom proximalen Ende 70 des Overtube 68 aus stellte sich bislang als problematisch dar.

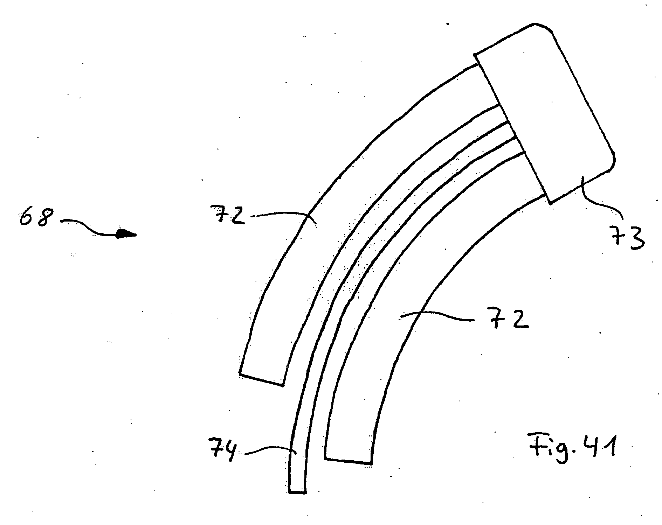

Zur Lösung dieses Problems weist der Overtube 68 ein zweites wellen- oder kabelförmiges Steuerelement 74 auf, das mit dem distalen Endelement 73 des Overtube 68 verbunden ist und bis zum proximalen Ende 70 des Overtube 68 reicht. Das zweite Steuerelement 74 stellt somit eine mechanische Einflussmöglichkeit auf das distale Endelement 73 des Overtube 68 vom proximalen Ende 70 des Overtube 68 dar. Zur Erhöhung der Flexibilität des Overtube 68 können wahlweise Rohrelemente 72 des Overtube 68 , in denen die elfte Vorrichtung 71 , beispielsweise ein Instrument 8 , geführt werden kann, von dem zweiten Steuerelement 74 derart entkoppelt werden, dass eine lokale Verschiebung eines Rohrelements 72 relativ zum zweiten Steuerelement 74 parallel zur Achse 78 des Overtube 68 möglich ist. Eine Krümmung des Overtube 68 führt damit nicht zu einer Stauchung in Richtung der Krümmung liegender Rohrelemente 72 bzw. Dehnung in Gegenrichtung der Krümmung liegender Rohrelemente 72, wodurch gegen die Krümmung wirkende Reaktionskräfte entstehen können, sondern zu einer lokalen Verschiebung der Rohrelemente 72 relativ zum zweiten Steuerelement 74 , (siehe Fig. 41).

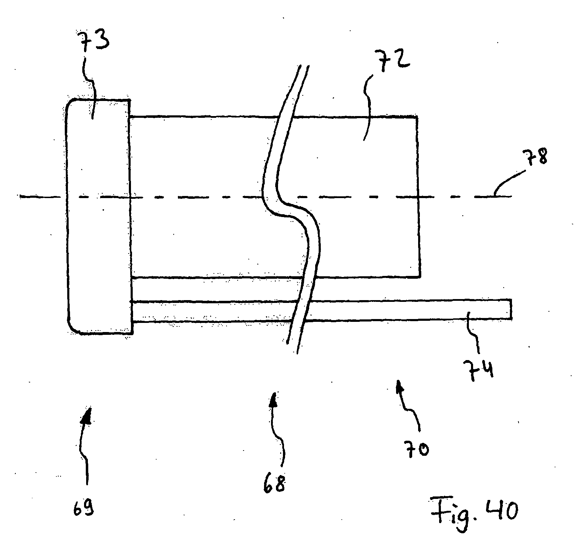

Der Overtube 68 weist konkreter beschrieben am distalen Ende 69 des Overtube 68 das distale Endelement 73 in Form einer Endkappe auf, das mit dem zweiten Steuerelement 74 verbunden ist. Das zweite Steuerelement 74 ist in der Form einer Welle oder eines Kabels ausgebildet und reicht bis zum proximalen Ende 70 des Overtube 68 . Des Weiteren weist der Overtube mindestens ein Rohrelement 72 auf, das mit dem distalen Endelement 73 verbunden ist (siehe Fig. 40).

Ein Beispiel für die Anwendung des Overtube 68 ist die Einführung eines Instruments 8 in das Rohrelement 72 . Das Instrument 8 stellt hierbei die elfte Vorrichtung 71 dar. Die Instrumentenspitze 80 des Instruments 8 schaut dabei vorzugsweise aus dem distalen Ende 69 des Overtube 68 heraus (siehe Fig. 25).

Die elfte Vorrichtung 71 ist parallel zur Achse 78 des Overtube verschieblich. Die Außenseite 116 der elften Vorrichtung 71 und die Innenseite 114 des Rohrelements 72 stehen dabei vorzugsweise in direktem Kontakt miteinander. Es kann vorteilhaft sein, die Reibung zwischen der Außenseite 116 der elften Vorrichtung 71 und der Innenseite 114 des Rohrelements 72 durch Verringerung der Kontaktfläche zu reduzieren. Des weiteren kann es vorteilhaft sein, die Querschnittsform der Außenseite 116 der elften Vorrichtung 71 und die Querschnittsform der Innenseite 114 des Rohrelements 72 derart zu gestalten, dass eine Rotation der elften Vorrichtung 71 im Rohrelement 72 blockiert wird.

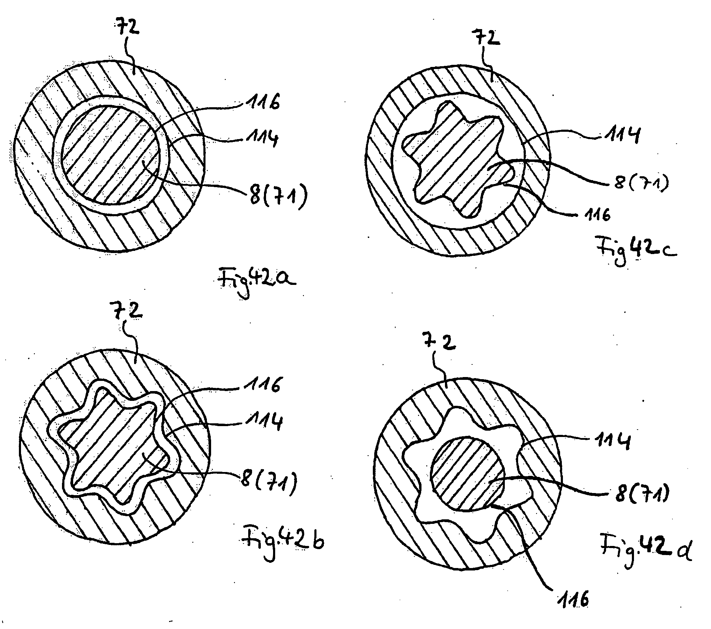

In einer vorteilhaften Ausgestaltung des Rohrelements 72 und der elften Vorrichtung 71 weist die Außenseite 116 der elften Vorrichtung 71 und die Innenseite 114 des Rohrelements 72 einen kreisrunden Querschnitt auf (siehe Fig. 42a). In einer weiteren vorteilhaften Ausgestaltung des Rohrelements 72 und der elften Vorrichtung 71 weisen die Außenseite 116 der elften Vorrichtung 71 und die Innenseite 114 des Rohrelements 72 Querschnittsformen auf, die eine Rotation der elften Vorrichtung 71 im Rohrelement 72 blockieren. Diese Querschnittsformen können beispielsweise ein Mehr-Eck sein oder sie können sternenförmig sein. Wahlweise weisen diese Querschnittsformen Rundungen auf (siehe Fig. 42b). In einer weiteren vorteilhaften Ausgestaltung des Rohrelements 72 und der elften Vorrichtung 71 weist die Innenseite 114 des Rohrelements 72 einen kreisrunden Querschnitt auf und weist die Außenseite 116 der elften Vorrichtung 71 eine Querschnittsform auf, die nicht kreisrund ist, beispielsweise ein Mehr-Eck oder sternenförmig. Wahlweise weist diese Querschnittsform Rundungen auf, (siehe Fig. 42c). In einer weiteren vorteilhaften Ausgestaltung des Rohrelements 72 und der elften Vorrichtung 71 weist die Außenseite 116 der elften Vorrichtung 71 einen kreisrunden Querschnitt auf, und weist die Innenseite 114 des Rohrelements 72 eine Querschnittsform auf, die nicht kreisrund ist, beispielsweise ein Mehr-Eck oder sternenförmig. Wahlweise weist diese Querschnittsform Rundungen auf (siehe Fig. 42d).

In einer vorteilhaften Ausgestaltung des Overtube 68 weist der Overtube 68 eine Führungsvorrichtung 117 auf. Diese Führungsvorrichtung 117 ist im vorliegenden Fall hülsenförmig ausgebildet, erstreckt sich längs des Overtube und ist mit dem mindestens einem Rohrelement 72 über die gesamte Rohrlänge fest verbunden. Innerhalb der hülsenförmigen Führungsvorrichtung ist das zweiten Steuerelement 74 axialverschieblich gelagert, so dass sich das Rohrelement 72 relativ zum zweiten Steuerelement 74 parallel zur Achse 78 des Overtube 68 lokal verschieben lässt. Vorzugsweise ist die Führungsvorrichtung 117 derart gestaltet, dass sich der Abstand zwischen der zweiten Steuervorrichtung 74 und dem Rohrelement 72 nicht verändern kann.

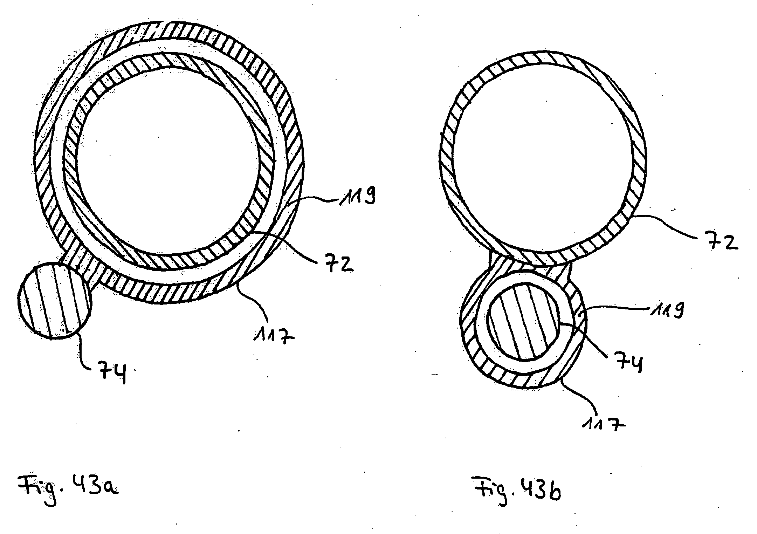

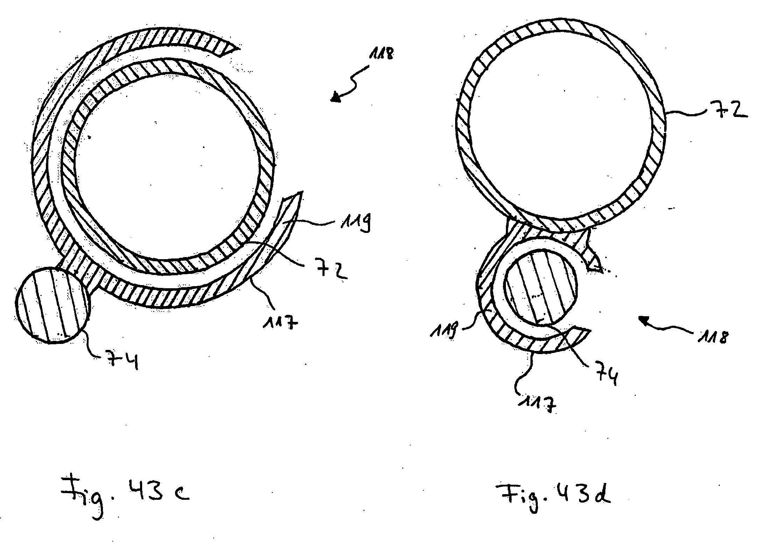

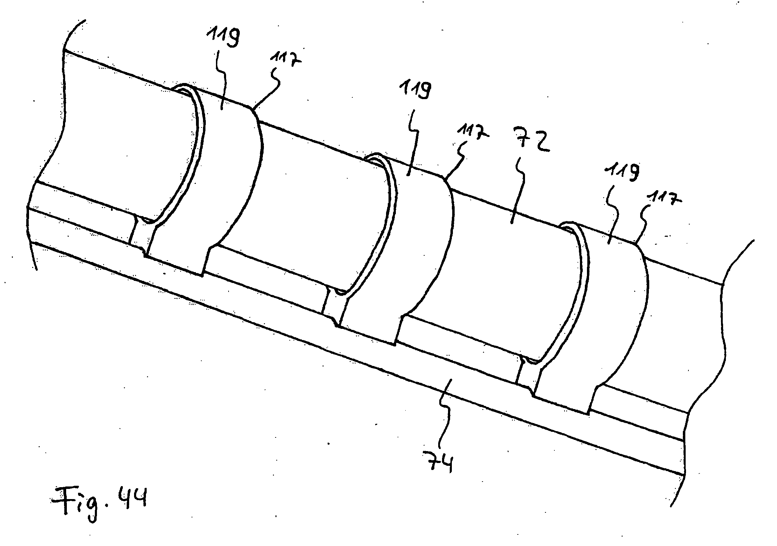

In einer vorteilhaften Ausgestaltung der Führungsvorrichtung 117 besteht diese aus mindestens einem rohr- oder hülsenförmigen Führungssegment 119 . Das Führungselement 119 muss nicht notwendiger Weise durchgehend über die gesamte Länge des Overtube verlaufen. Vorzugsweise können mehrere Führungssegmente oder Hülsen 119 in regelmäßigen Abständen längs des Overtube angeordnet sein (siehe Fig. 44) die alle mit dem jeweiligen Rohrelement fest verbunden sind. In einer anderen vorteilhaften Ausgestaltung des Führungssegments 119 ist dieses fest mit dem zweiten Steuerelement 74 verbunden und weist eine geschlossene Ringstruktur auf, in der das Rohrelement 72 verschiebbar geführt ist (siehe Fig. 43a). In einer weiteren vorteilhaften Ausgestaltung des Führungssegments 119 ist dieses fest mit dem Rohrelement 72 verbunden und weist eine geschlossene Ringstruktur auf, in der das zweite Steuerelement 74 verschiebbar geführt ist (siehe Fig. 43b). In einer weiteren vorteilhaften Ausgestaltung des Führungssegments 119 ist dieses fest mit dem zweiten Steuerelement 74 verbunden und weist eine Ringstruktur auf, die eine seitliche Öffnung 118 aufweist und in der das Rohrelement 72 verschiebbar geführt ist (siehe Fig. 43c). In einer weiteren vorteilhaften Ausgestaltung des Führungssegments 119 ist dieses fest mit dem Rohrelement 72 verbunden und weist eine Ringstruktur auf, die eine seitliche Öffnung 118 aufweist und in der das zweite Steuerelement 74 verschiebbar geführt ist (siehe Fig. 43d).

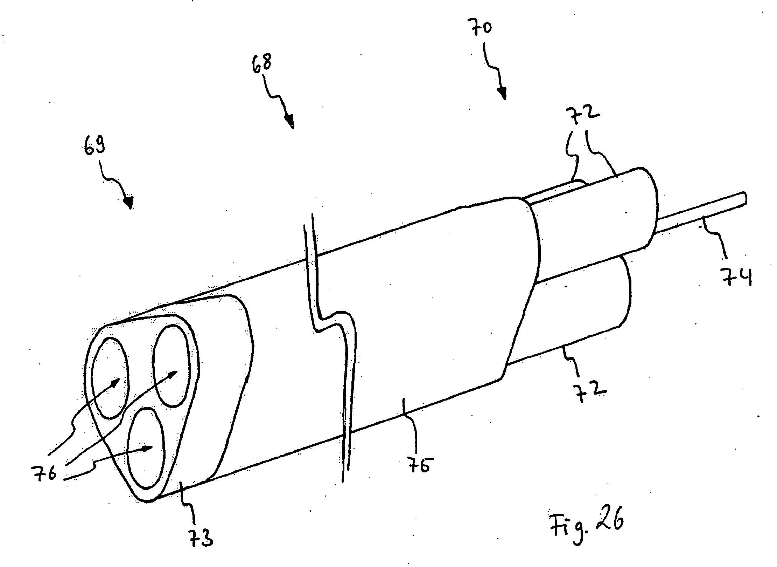

In einem Beispiel für die Ausführung des Overtube 68 weist der Overtube 68 drei Rohrelemente 72 auf. Das distale kappenförmige Endelement 73 weist drei distale Öffnungen 76 auf, die jeweils eine Verbindung zu einem der Rohrelemente 72 herstellen. Die Rohrelemente 72 sind mit dem distalen Endelement 73 verbunden und werden durch dieses zusammengehalten. Zwei der Rohrelemente 72 sind dabei vorzugsweise für die Aufnahme jeweils eines Instruments 8 vorgesehen, ein weiteres Rohrelement 72 ist dabei vorzugsweise für die Aufnahme eines flexiblen Endoskops 113 vorgesehen (siehe Fig. 26).

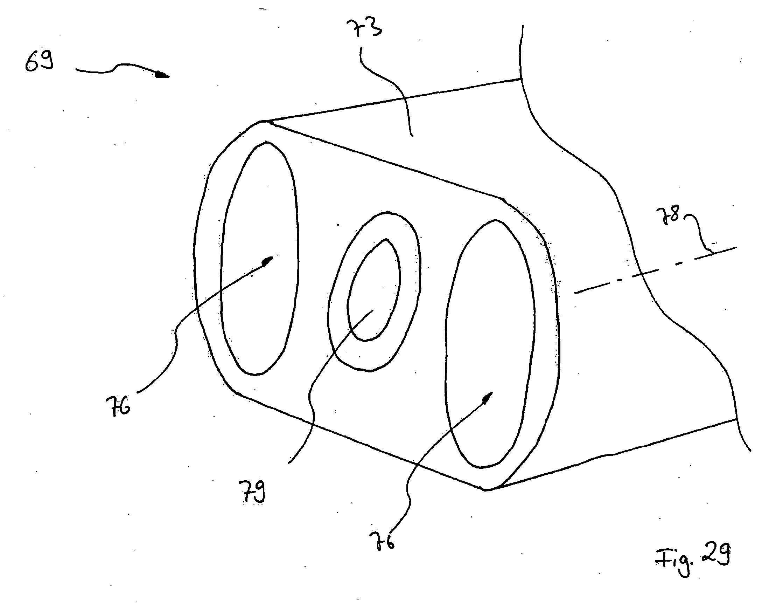

In einem weiteren Beispiel für die Ausführung des Overtube 68 weist der Overtube 68 zwei Rohrelemente 72 auf. Das distale Endelement 73 weist zwei distale Öffnungen 76 auf, die jeweils eine Verbindung zu einem der Rohrelemente 72 herstellen. Die Rohrelemente sind mit dem distalen Endelement 73 verbunden und werden durch dieses zusammengehalten. Die zwei Rohrelemente 72 sind dabei vorzugsweise für die Aufnahme jeweils eines Instruments 8 vorgesehen. Das distale Endelement 73 weist eine integral darin angeordnete Kameraeinheit 79 auf (siehe Fig. 29).

In einer vorteilhaften Ausgestaltung der Kameraeinheit 79 weist die Kameraeinheit 79 eine mechanische Vorrichtung oder Antrieb auf, über die eine Einstellung des Blickwinkels der Kameraeinheit 79 erfolgen kann.

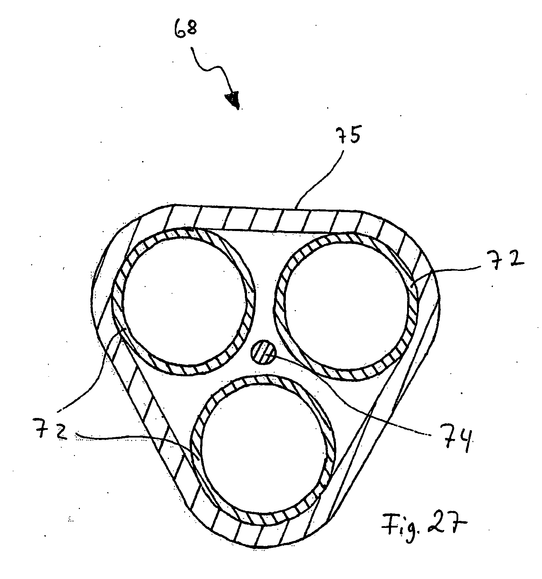

In einer vorteilhaften Ausgestaltung des Overtube 68 weist der Overtube 68 eine äußere Hülle 75 auf, welche die jeweiligen parallel laufenden Rohrelemente 72 bündelt (siehe Fig. 27).

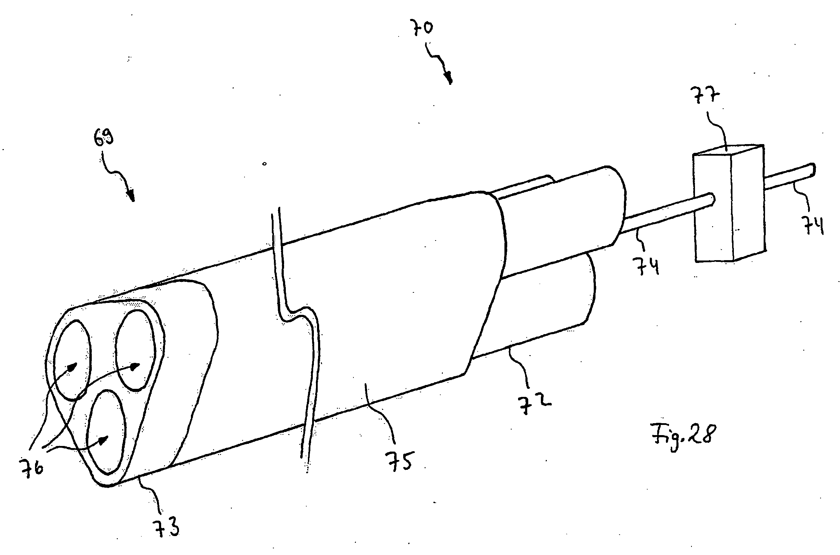

In einer vorteilhaften Ausgestaltung des zweiten kabelförmigen Steuerelements 74 ist das zweite Steuerelement 74 am proximalen Ende mit einem vierten Bedienelement 77 verbunden. Das kabbelförmige Steuerelement 74 weist hierbei ein vorbestimmte Torsions- und Biegesteifigkeit ähnlich einer Bowdenzugeinrichtung auf. Die Verbindung zwischen dem zweiten Steuerelement 74 und dem vierten Bedienelement 77 ist dabei derart gestaltet, dass eine Rotation des vierten Bedienelements 77 eine Rotation des zweiten Steuerelements 74 bewirkt und eine Verschiebung des vierten Bedienelements 77 eine Verschiebung des zweiten Steuerelements 74 bewirkt. Durch Betätigen des vierten Bedienelements 77 kann damit die Ausrichtung und Position des distalen Endelements 73 kontrolliert werden (siehe Fig. 28).

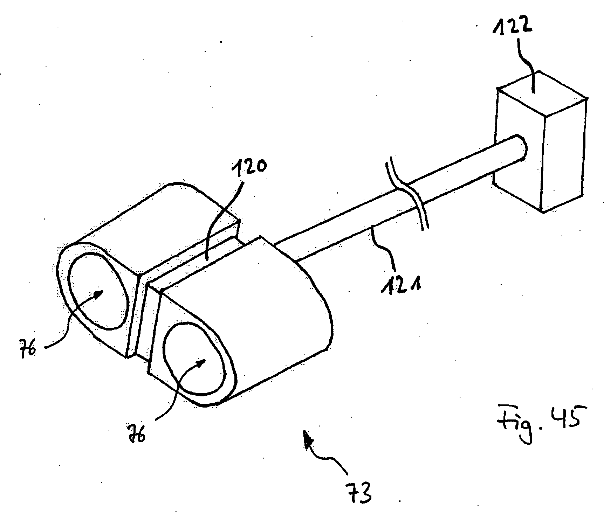

In einer vorteilhaften Ausgestaltung des distalen Endelements 73 weist dieses mindestens eine distale Öffnung 76 sowie eine Aktuierungsvorrichtung 120 auf, durch die die Position oder die Ausrichtung oder sowohl die Position als auch die Ausrichtung der distalen Öffnung 76 eingestellt werden kann. Die Aktuierungsvorrichtung 120 weist ein drittes Steuerelement 121 und ein achtes Bedienelement 122 auf, wobei das dritte Steuerelement 121 mit dem achten Bedienelement 122 verbunden ist (siehe Fig. 45).

In einer vorteilhaften Weiterbildung der Aktuierungsvorrichtung 120 weist die Aktuierungsvorrichtung 120 einen pneumatischen Aktuator auf, der bei Beaufschlagung mit Druckluft die Position oder die Ausrichtung oder sowohl die Position als auch die Ausrichtung einer distalen Öffnung 76 einstellen kann. Die Beaufschlagung mit Druckluft erfolgt über das dritte Steuerelement 121 . Die Steuerung der Beaufschlagung mit Druckluft erfolgt über das achte Bedienelement 122 .

In einer weiteren vorteilhaften Weiterbildung der Aktuierungsvorrichtung 120 weist die Aktuierungsvorrichtung 120 einen hydraulischen Aktuator auf, der bei Einpumpen bzw. Abpumpen eines flüssigen Mediums die Position oder die Ausrichtung oder sowohl die Position als auch die Ausrichtung einer distalen Öffnung 76 einstellen kann. Das Einpumpen bzw. Abpumpen eines flüssigen Mediums erfolgt über das dritte Steuerelement 121 . Die Steuerung des Einpumpens bzw. Abpumpens eines flüssigen Mediums erfolgt über das achte Bedienelement 122 .

In einer weiteren vorteilhaften Weiterbildung der Aktuierungsvorrichtung 120 weist die Aktuierungsvorrichtung 120 ein mechanisches Getriebe auf, das bei Einkoppelung einer Kraft bzw. eines Drehmoments die Position oder die Ausrichtung oder sowohl die Position als auch die Ausrichtung einer distalen Öffnung 76 einstellen kann. Die Einkoppelung einer Kraft bzw. eines Drehmoments erfolgt über das dritte Steuerelement 121 . Die Steuerung der Einkoppelung einer Kraft bzw. eines Drehmoments erfolgt über das achte Bedienelement 122 .

In einer vorteilhaften Weiterbildung des Rohrelements 72 weist das Rohrelement 72 einen Mechanismus auf, der eine Verschiebung einer in dem Rohrelement 72 befindlichen elften Vorrichtung 71 , vorzugsweise eines Instruments 8 oder eines flexiblen Endoskops 113 , blockieren kann. Dadurch kann eine bevorzugte Position der elften Vorrichtung 71 in dem Rohrelement 72 von dem Rohrelement 72 festgehalten werden.

In einer weiteren vorteilhaften Weiterbildung des Rohrelements 72 weist das Rohrelement 72 einen Mechanismus auf, der eine Rotation einer in dem Rohrelement 72 befindlichen elften Vorrichtung 71 , vorzugsweise eines Instruments 8 oder eines flexiblen Endoskops 113 , blockieren kann. Dadurch kann eine bevorzugte Ausrichtung der elften Vorrichtung 71 in dem Rohrelement 72 von dem Rohrelement 72 festgehalten werden.

In einer beispielhaften Gestaltung der distalen Öffnung 76 des distalen Endelements 73 weist die distale Öffnung 76 ein Schlauchelement 123 auf. Das Schlauchelement 123 ist derart an dem distalen Endelement 73 befestigt, dass eine in ein Rohrelement 72 eingeführte elfte Vorrichtung 71 am distalen Ende 69 des Overtube 68 aus der distalen Öffnung 76 austreten kann. Durch die Verwendung eines Schlauchelements aus einem flexiblen Material, beispielsweise einer Kunststofffolie, kann der Gesamtquerschnitt des distalen Endes 69 des Overtube 68 reduziert werden, denn das Lumen der distalen Öffnung 76 kann, sofern sich keine elfte Vorrichtung 71 in der distalen Öffnung befindet, kollabieren und den Querschnitt des distalen Endes 69 des Overtube 68 damit reduzieren. Dies ist insbesondere bei der Einführung des Overtube 68 in ein röhrenförmiges Hohlorgan sinnvoll, da ein kleiner Querschnitt eine einfache und schonende Einführung des Overtube 68 begünstigen kann. Das Lumen der distalen Öffnung 76 kann durch Einführung einer elften Vorrichtung 71 aufgeweitet werden.

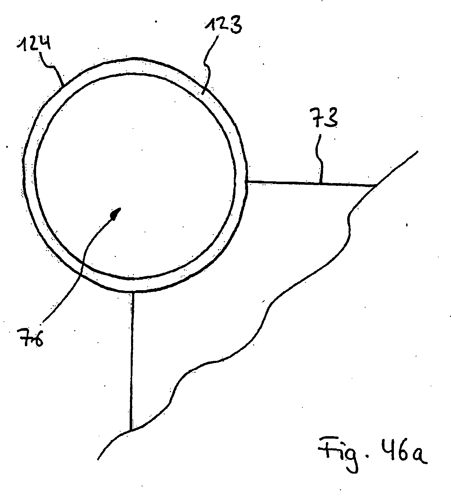

In einer vorteilhaften Weiterbildung des Schlauchelements 123 weist das Schlauchelement 123 einen geschlossenen Querschnitt auf und ist an einem Abschnitt der Außenfläche 124 des Schlauchelements 123 mit dem distalen Endelement 73 verbunden, (siehe Fig. 46a).

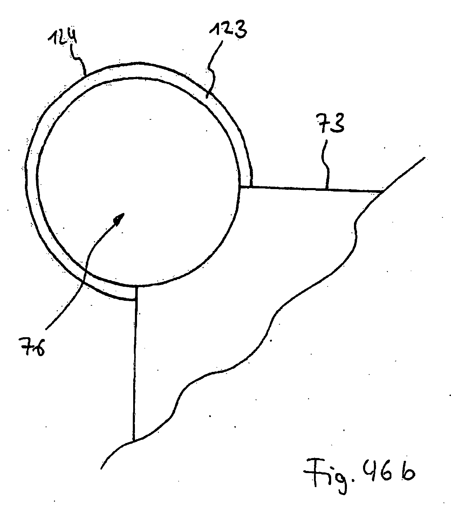

In einer weiteren vorteilhaften Weiterbildung des Schlauchelements 123 weist das Schlauchelement 123 einen offenen Querschnitt auf und ist derart mit dem distalen Endelement 73 verbunden, dass die entstehende distale Öffnung 76 einen geschlossenen Querschnitt aufweist, (siehe Fig. 46b).

In einer vorteilhaften Weiterbildung des Rohrelements 72 besteht das Rohrelement 72 ganz oder teilweise aus einem flexiblen Material, beispielsweise einer Kunststofffolie, die das Kollabieren des Lumens des Rohrelements 72 zulässt, sofern sich keine elfte Vorrichtung 71 in dem Rohrelement 72 befindet. Dadurch kann eine Reduzierung des Querschnitts des Overtube 68 erreicht werden. Dies ist insbesondere bei der Einführung des Overtube 68 in ein röhrenförmiges Hohlorgan sinnvoll, da ein kleiner Querschnitt eine einfache und schonende Einführung des Overtube 68 begünstigen kann. Das Lumen des Rohrelements 72 kann durch Einführung einer elften Vorrichtung 71 aufgeweitet werden.

Die Flexibilität des Overtube 68 wird teilweise bestimmt durch das zweite Steuerelement 74 . Während der Einführung des Overtube 68 in ein Hohlorgan ist eine möglichst hohe Flexibilität wünschenswert. Hat das distale Ende den Interventionsort erreicht, kann dagegen eine geringe Flexibilität des Overtube 68 wünschenswert sein, um eine gute Kontrollierbarkeit des distalen Endelements 73 zu erreichen.

In einer vorteilhaften Weiterbildung des zweiten Steuerelements 74 weist das zweite Steuerelement 74 einen Mechanismus auf, durch den die Flexibilität des gesamten zweiten Steuerelements 74 wahlweise eingestellt werden kann.

In einer weiteren vorteilhaften Weiterbildung des zweiten Steuerelements 74 weist das zweite Steuerelement 74 einen Mechanismus auf, durch den die Flexibilität mindestens eines Abschnittes des zweiten Steuerelements 74 wahlweise eingestellt werden kann.

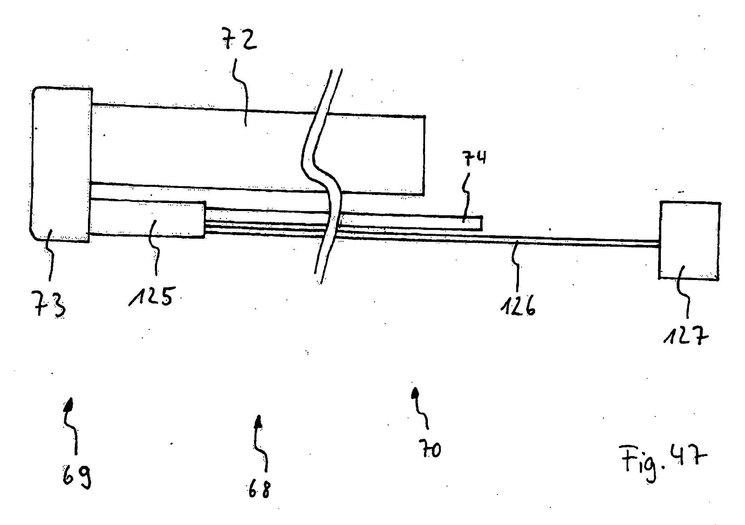

In einer weiteren vorteilhaften Weiterbildung des zweiten Steuerelements 74 weist das zweite Steuerelement 74 ein Steuerungssegment 125 auf. Das Steuerungssegment 125 des zweiten Steuerelements 74 weist ein viertes Steuerelement 126 und ein neuntes Bedienelement 127 auf. Das neunte Bedienelement 127 befindet sich vorzugsweise am proximalen Ende 70 des Overtube 68 und ist über das vierte Steuerelement 126 mit des Steuerungssegment 125 verbunden. Das Steuerungssegment 125 des zweiten Steuerelements 74 befindet sich vorzugsweise am distalen Ende 69 des Overtube 68 , (siehe Fig. 47).

Das Steuerungssegment 125 ist derart ausgeführt, dass bei Betätigung des neunten Bedienelements 127 über das vierte Steuerelement 126 eine Einstellung der Beugung des Steuerungssegments 125 erfolgen kann. Durch die Einstellung der Beugung des Steuerungssegments 125 kann vorzugsweise die Ausrichtung des distalen Endelements 73 des Overtube 68 eingestellt werden.

Des Weiteren ist eine Stabilisierung des distalen Endes 69 des Overtube 68 vorteilhaft, insbesondere bei Manipulation des Zielgewebes mit chirurgischen Instrumenten, die aus den distalen Öffnungen 76 des Overtube 68 herausgeführt sind. Eine solche Stabilisierung kann durch Abstützen des Overtube 68 an der Hohlorganwand 129 erfolgen. Insbesondere in einem röhrenförmigen Hohlorgan mit geringen Schwankungen der Querschnittsfläche wie dem Dickdarm kann eine solche Stabilisierung des distalen Endes 69 des Overtube 68 erfolgen.

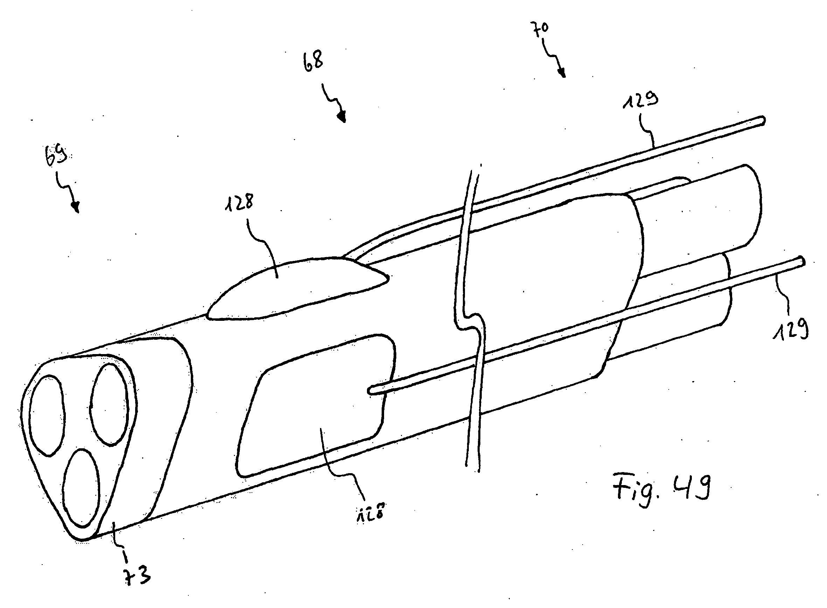

In einer vorteilhaften Weiterbildung des Overtube 68 weist der Overtube 68 an der Außenseite mindestens eine erste Fluidkammer 128 auf. Über eine Fluidzuführung 129 kann ein Fluid in die erste Fluidkammer 128 eingebracht bzw. aus der ersten Fluidkammer 128 entlassen werden. Durch Einbringen eines Fluids in die erste Fluidkammer 128 kann der Querschnitt des Overtube 68 selektiv vergrößert werden, (siehe Fig. 49).

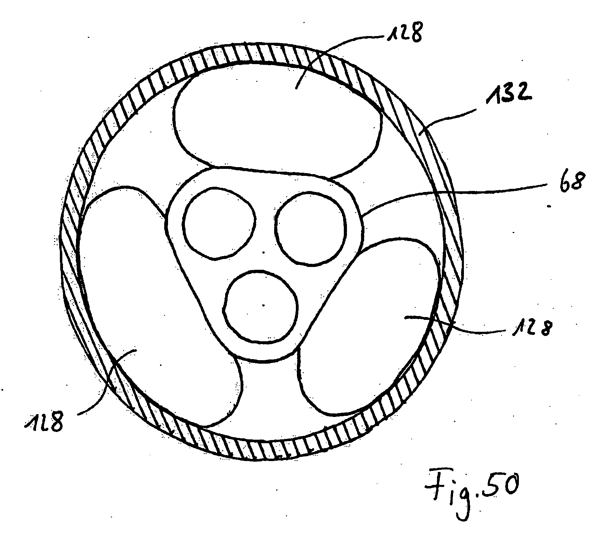

Durch die Einbringung eines Fluids in die erste Fluidkammer 128 kann eine Stabilisierung des distalen Endes 69 des Overtube 68 in einem Hohlorgan durch Abstützen des Overtube 68 an der Hohlorganwand 129 erreicht werden, (siehe Fig. 50).

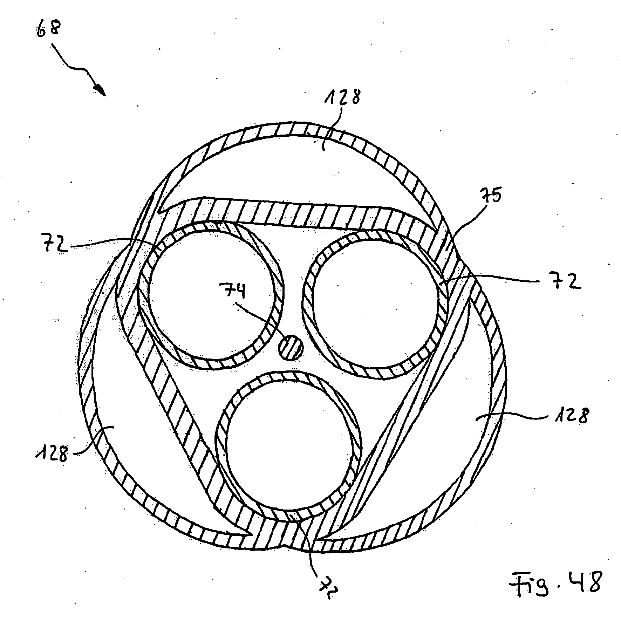

In einer vorteilhaften Weiterbildung der äußeren Hülle 75 weist die äußere Hülle 75 mindestens eine erste Fluidkammer 128 auf, (siehe Fig. 48).

In einer vorteilhaften Weiterbildung des Overtube 68 weist der Overtube eine kollabierbare Struktur auf. Hierzu können beispielsweise die Rohrelemente 72 und die äußere Hülle 75 aus einem flexiblen Material bestehen, vorzugsweise einer Kunststofffolie. Dies kann die Einführung des Overtube 68 in den menschlichen Körper einfacher und schonender gestalten.

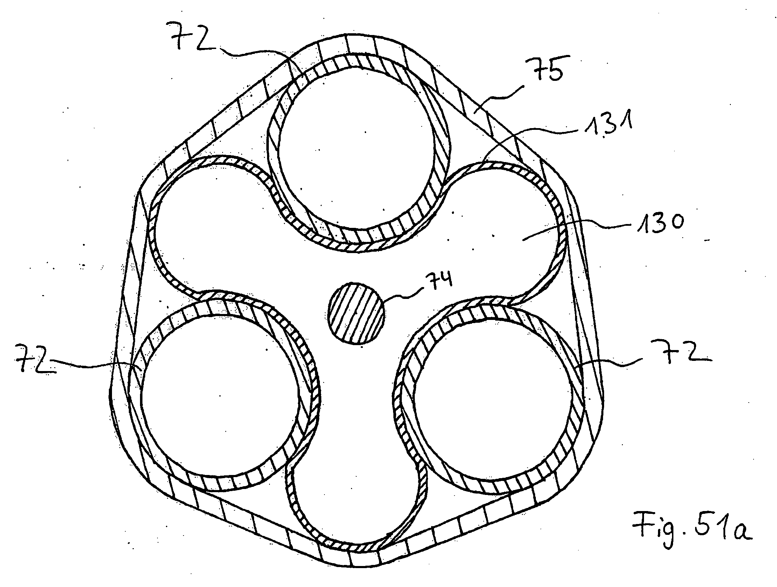

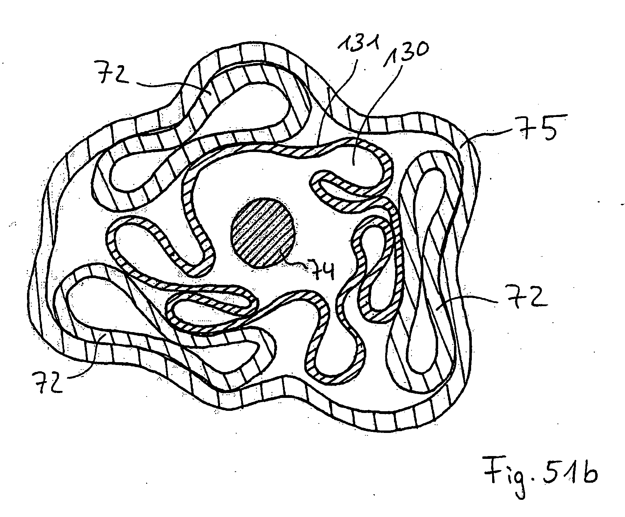

In einer weiteren vorteilhaften Weiterbildung des Overtube 68 bei der der Overtube 68 eine kollabierbare Struktur aufweist, weist der Overtube 68 ein Fluidkammersystem 131 bestehend aus mindestens einer zweiten Fluidkammer 130 auf, das bei Befüllen mit einem Fluid eine stützende Struktur darstellt. Diese stützende Struktur kann dazu dienen, den kollabierten Querschnitt des Overtube 68 aufzurichten und beispielsweise die Einführung eines Instruments 8 in die Rohrelemente 72 zu erleichtern. Wenn das Fluidkammersystem 131 unzureichend mit Fluid befüllt ist, kann die Struktur kollabieren, (siehe Fig. 51b). Wenn das Fluidkammersystem 131 ausreichend mit Fluid befüllt, wird die Einstellung einer bevorzugten Querschnittsform des Overtube 68 begünstigt, (siehe Fig. 51a). Das Fluid kann hierbei wahlweise ein Gas oder eine Flüssigkeit sein.

In einer weiteren vorteilhaften Weiterbildung des Overtube 68 bei der der Overtube 68 ein Fluidkammersystem 131 zur Stützung einer kollabierbaren Struktur bestehend aus mindestens einer zweiten Fluidkammer 130 aufweist, ist das Fluidkammersystem 131 in Segmente unterteilt, die vorzugsweise in regelmäßigen Abständen entlang des Obertube 68 angeordnet sind. Wahlweise können die Segmente des Fluidkammersystems 131 selektiv mit Fluid befüllt werden. Durch die segmentweise Ausführung des Fluidkammersystems 131 kann bei Befüllen des Fluidkammersystems 131 mit einem Fluid eine Aufrechterhaltung einer Biegung des Overtube 68 begünstigt werden. Das Fluid kann hierbei wahlweise ein Gas oder eine Flüssigkeit sein.

Der Overtube 68 kann einen symmetrischen, aber auch einen asymmetrischen Querschnitt aufweisen. Insbesondere in dem Fall, dass die in die Rohrelementen 72 einzuführenden medizinischen Instrumente unterschiedliche Durchmesser aufweisen, ist eine asymmetrische Ausgestaltung des Querschnitts des Overtube durch die Verwendung unterschiedlich großer Rohrelemente 72 sinnvoll.
- (1): Inneres Rohrelement
- (2): Äußeres Rohrelement
- (3): Erste Strukturen
- (4): Zugelement
- (5): Schaft / Instrumentenschaft
- (6): Proximales Ende der ersten Vorrichtung / Proximales Ende des Instruments
- (7): Distales Ende der ersten Vorrichtung
- (8): Erste Vorrichtung / Instrument
- (9): Achse der ersten Vorrichtung / Achse des Instruments
- (10): Distale Öffnung des inneren Rohrelements
- (11): Seitliche Aussparung
- (12): Außenseite der seitlichen Aussparung
- (13): Innenseite der seitlichen Aussparung
- (14): Schnittflächen des Freiraums
- (15): Stirnfläche des inneren Rohrelements
- (16): Segment
- (17): Scharnierelement
- (18): Schlaufe des Zugelements
- (19): Durchgehende Öffnung des Segments
- (20): Rotationsachse des Scharnierelements
- (21): Linie tangential zur Außenseite des Segments
- (22): Achse des Segments
- (23): Biegeelement
- (24): Schaft des inneren Rohrelements
- (25): Freiraum
- (26): Biegebereich
- (27): Verjüngung
- (28): Zweite Zugelementführung
- (29): Erste mechanische Verbindung
- (30): Zweite mechanische Verbindung
- (31): Seitliche Öffnung im äußeren Rohrelement
- (32): Proximale Verdickung im Zugelement
- (33): Seitliche Öffnung im inneren Rohrelement
- (34): Distale Verdickung im Zugelement
- (35): Erste Zugelementführung
- (36): Führungselement
- (37): Nut in der Innenseite des inneren Rohrelements
- (38): Durchgehende Bohrung in der Außenwand des inneren Rohrelements
- (39): Mechanisches Getriebeelement
- (40): Zweite seitliche Öffnung in der Außenwand des inneren Rohrelements
- (41): Außenseite des äußeren Rohrelements
- (42): Innenseite des äußeren Rohrelements
- (43): Außenseite des Schaftes des inneren Rohrelements
- (44): Innenseite des Schaftes des inneren Rohrelements
- (45): Zweite Vorrichtung
- (46): Fünftes Bedienelement der zweiten Vorrichtung
- (47): Schaft der zweiten Vorrichtung
- (48): Chirurgischer Effektor
- (49): Proximales Ende des flexiblen chirurgischen Instruments
- (50): Distales Ende des flexiblen chirurgischen Instruments
- (51): Außenseite der zweiten Vorrichtung
- (52): Vierte Vorrichtung
- (53): Erstes Steuerelement
- (54): Erstes Verbindungselement
- (55): Fünfte Vorrichtung / Steuervorrichtung
- (56): Sechste Vorrichtung
- (57): Siebte Vorrichtung
- (58): Endelement
- (59): Erstes Bedienelement
- (60): Erstes Element der sechsten Vorrichtung
- (61): Zweites Element der sechsten Vorrichtung
- (62): Achte Vorrichtung
- (63): Zweites Bedienelement
- (64): Drittes Element der sechsten Vorrichtung
- (65): Neunte Vorrichtung
- (66): Drittes Bedienelement
- (67): Achse der sechsten Vorrichtung
- (68): Zehnte Vorrichtung / Overtube
- (69): Distales Ende der zehnten Vorrichtung
- (70): Proximales Ende der zehnten Vorrichtung
- (71): Elfte Vorrichtung
- (72): Rohrelement / Instrumentenkanal
- (73): Distales Endelement
- (74): Zwölfte Vorrichtung / Zweites Steuerelement
- (75): Äußere Hülle
- (76): Distale Öffnungen
- (77): Viertes Bedienelement
- (78): Achse der zehnten Vorrichtung
- (79): Kameraeinheit
- (80): Instrumentenspitze
- (81): Vorzugsrichtung
- (82): Distales Ende des Zugelements
- (83): Proximales Ende des Zugelements
- (84): Nut in der Außenseite des inneren Rohrelements
- (85): Chirurgisches Instrument
- (86): Chirurgischer Effektor
- (87): Zweites Steuerelement / Kraftübertragungselement
- (88): Erstes Stirnzahnrad
- (89): Zweites Stirnzahnrad
- (90): Erstes Kegelzahnrad
- (91): Zweites Kegelzahnrad
- (92): Zweites Verbindungselement
- (93): Griff
- (94): Führungselement
- (95): Drehvorrichtung
- (96): Zweites Kraftübertragungselement
- (97): Bevorzugte Drehrichtung
- (98): Federelement
- (99): Drittes Verbindungselement
- (100): Griff
- (101): Führungselement
- (102): Umlenkvorrichtung
- (103): Kraftübertragungselement
- (104): Drehvorrichtung
- (105): Vorzugsrichtung
- (106): Umlenkvorrichtung
- (107): Führung
- (108): Federelement
- (109): Viertes Verbindungselement
- (110): Griff
- (111): Siebtes Bedienelement
- (112): Sechstes Bedienelement
- (113): Flexibles Endoskop
- (114): Innenseite des Rohrelements
- (115): Außenseite des Rohrelements
- (116): Außenseite der elften Vorrichtung
- (117): Führungsvorrichtung
- (118): Seitliche Öffnung der Führungsvorrichtung
- (119): Führungssegmente
- (120): Aktuierungsvorrichtung
- (121): Drittes Steuerelement
- (122): Achtes Bedienelement
- (123): Schlauchelement
- (124): Außenfläche des Schlauchelements
- (125): Steuerungssegment des zweiten Steuerelements
- (126): Viertes Steuerelement
- (127): Neuntes Bedienelement
- (128): Erste Fluidkammer
- (129): Hohlorganwand
- (130): Zweite Fluidkammer
- (131): Fluidkammersystem

Fig. 1: Erste Vorrichtung
Fig. 2: Erste Strukturen bestehend aus seitlichen Aussparungen
Fig. 3: Vorteilhafte Weiterbildungen der seitlichen Aussparungen
Fig. 4: Vorteilhafte Weiterbildungen der ersten Strukturen
Fig. 5: Erste Strukturen bestehend aus Segmenten und Scharnierelementen
Fig. 6: Vorteilhafte Positionierungen des Scharnierelements
Fig. 7: Erste Strukturen bestehend aus Segmenten und einem Biegeelement
Fig. 8: Biegebereich des Biegeelements
Fig. 9: Vorteilhafte Anordnung des / der Biegeelements/e
Fig. 10: Vorteilhafte Querschnittsformen des Biegeelements
Fig. 11: Funktion des Zugelements
Fig. 12: Vorteilhafte Weiterbildungen der ersten mechanischen Verbindung
Fig. 13: Vorteilhafte Weiterbildungen der zweiten mechanischen Verbindung
Fig. 14: Zweite Führung des Zugelements
Fig. 15: Vorteilhafte Weiterbildungen des Führungselements
Fig. 16: Vorteilhafte Weiterbildungen der zweiten Führung des Zugelements
Fig. 17: Vorteilhafte Weiterbildung der zweiten Führung des Zugelements
Fig. 18: Vorteilhafte Weiterbildungen des Querschnitts des inneren und äußeren Rohrelements
Fig. 19: Ausführung der zweiten Vorrichtung
Fig. 20: Vorteilhafte Weiterbildungen des Querschnitts der zweiten Vorrichtung und des inneren Rohrelements
Fig. 21: Vorteilhafte Weiterbildungen der zweiten Vorrichtung
Fig. 22: Vorteilhafte Ausführung der fünften Vorrichtung
Fig. 23: Vorteilhafte Ausführung der fünften Vorrichtung
Fig. 24: Vorteilhafte Ausführung der fünften Vorrichtung
Fig. 25: Zehnte Vorrichtung
Fig. 26: Vorteilhafte Ausführung der zehnten Vorrichtung
Fig. 27: Querschnittsdarstellung einer vorteilhaften Ausführung der zehnten Vorrichtung
Fig. 28: Vorteilhafte Ausführung der zehnten Vorrichtung mit viertem Bedienelement
Fig. 29: Vorteilhafte Ausführung des distalen Endelements der zehnten Vorrichtung mit integrierter Kameraeinheit

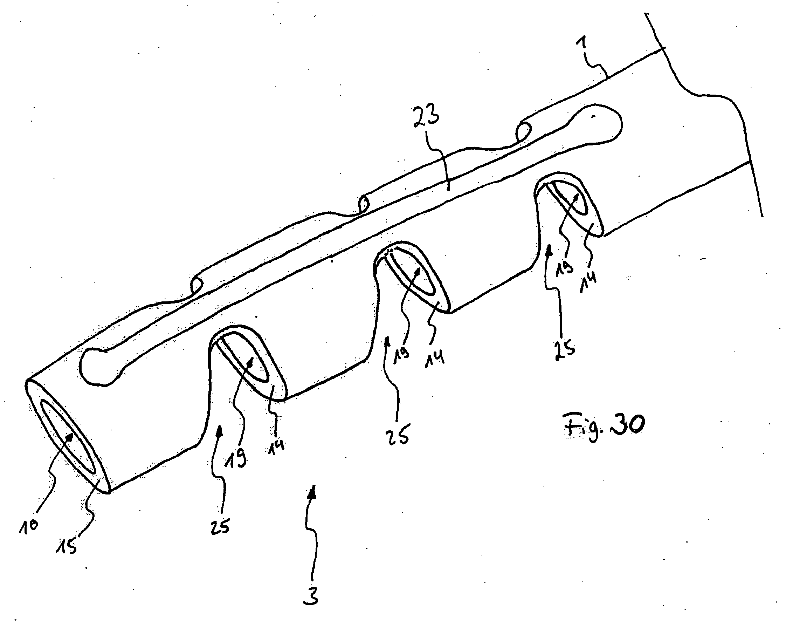

Fig. 30: Vorteilhafte Ausführung der ersten Strukturen
Fig. 31: Vorteilhafte Ausführungen der vierten Vorrichtung
Fig. 32: Vorteilhafte Ausführung des ersten Bedienelements
Fig. 33: Vorteilhafte Ausführung des ersten Bedienelements
Fig. 34: Vorteilhafte Ausführung der siebten Vorrichtung
Fig. 35: Vorteilhafte Ausführung des zweiten Bedienelements
Fig. 36: Vorteilhafte Ausführung des ersten Bedienelements und des zweiten Bedienelements
Fig. 37: Vorteilhafte Ausführung der achten Vorrichtung
Fig. 38: Vorteilhafte Ausführungen der zweiten Vorrichtung
Fig. 39: Beispielhafte Ausführung der zweiten Vorrichtung
Fig. 40: Overtube
Fig. 41: Funktion des zweiten Steuerelements bei Biegung des Overtube
Fig. 42: Vorteilhafte Ausführungen des Rohrelements und er elften Vorrichtung
Fig. 43: Vorteilhafte Ausführungen der Führungsvorrichtung
Fig. 44: Vorteilhafte Ausführungen der Führungssegmente
Fig. 45: Aktuierungsvorrichtung
Fig. 46: Vorteilhafte Ausführungen des Schlauchelements
Fig. 47: Vorteilhafte Ausführung des zweiten Steuerelements
Fig. 48: Vorteilhafte Ausführung der äußeren Hülle
Fig. 49: Vorteilhafte Ausführung des Overtube
Fig. 50: Vorteilhafte Anwendung der Fluidkammern
Fig. 51: Funktion des Fluidkammersystems

## Patentansprüche

1. Einführhilfe für medizinische Instrumente bestehend aus einem Schaft (5) mit einem distalen Ende (7), an welchem ein Abwinkelstück mit eine Abwinklung in zumindest eine Vorzugsrichtung (81) begünstigenden Strukturen (3) unterschiedlich zum Schaft (5) angeordnet ist, sowie einem proximalen Ende (6), an dem eine Handhabe zur manuellen Steuerung des Abwinkelstücks für dessen Abwinkeln um einen bestimmten Winkel angeordnet ist und einem Zugelement (4) zur Betätigung des Abwinkelstücks, wobei der Schaft (5) zwischen der Handhabe und dem Abwinkelstück aus einem äußeren, sich bis zum Bereich des distalen Endes (7) des Schafts (5) erstreckenden Rohrelement (2) und einem im äußeren Rohrelement (2) geführten sowie im Bereich des distalen Endes (7) aus dem äußeren Rohrelement (2) herausragenden inneren Rohrelement (1) besteht, und das innere Rohrelement (1) an seinem distalen Ende das Abwinkelstück aufweist, an dessen distalem Ende das Zugelement (4) dezentral befestigt ist, **dadurch gekennzeichnet, dass** das Zugelement (4) am distalen Ende des äußeren Rohrelementes angelenkt ist.

2. Einführhilfe nach Anspruch 1, **dadurch gekennzeichnet, dass** die Strukturen des Abwinkelstücks aus einer Anzahl von längs beabstandeten äußeren Einkerbungen oder Spalte in der Wand des inneren Rohrelements besteht.

3. Einführhilfe nach Anspruch 1, **dadurch gekennzeichnet, dass** die Strukturen durch eine Anzahl von längs beabstandeten Rohrstücke oder Glieder gebildet wird, die über Scharniere miteinander sowie mit dem inneren Rohrelement derart verbunden sind, dass eine fortlaufende Kette aus Rohrstücken oder Gliedern entsteht.

4. Einführhilfe nach einem der vorstehenden Ansprüche **dadurch gekennzeichnet, dass** das Zugelement durch an den Rohrstücken oder Gliedern ausgebildeten Führungsbohrungen hindurchführend zum distalen Ende des äußeren Rohrelements für eine Zug- und/oder Druckkraftübertragung auf das distale Ende des Abwinkelstücks zurückgeführt ist.

5. Einführhilfe nach Anspruch 1, **dadurch gekennzeichnet, dass** die Handhabe oder der Schaft eine Arretierung hat, um eine bestimmte Winkelposition des Abwinkelstücks zu halten.

6. Einführhilfe gemäß einem der Ansprüche 1 bis 5 mit zwei Abzugsbügeln oder - griffen, die an ihren einen Enden mit jeweils einem der distalen Enden des inneren und äußeren Rohrelements für eine Relatiwerschiebung der beiden Rohrelemente in axialer Richtung verbindbar sind.

7. Einführhilfe nach Anspruch 6 **gekennzeichnet durch** eine Arretiervorrichtung zur Arretierung der aktuellen Betätigungsposition der Handhabe und damit der Winkelstellung des Abwinkelstücks.

8. Einführhilfe nach einem der Ansprüche 6 und 7 **gekennzeichnet durch** eine Nullpunktseinstellung zur Einstellung einer Ausgangsrelativlage der beiden Rohrelemente in einer Ausgangstellung der Handhabe.

9. Einführhilfe nach einem der Ansprüche 1 bis 8, **dadurch gekennzeichnet, dass** sie eine Abzugsbügel oder-griff aufweist, der mit einem in der Einführhilfe liegenden oder an der Einführhilfe befestigten medizinischen Instrument für eine axiale Verschiebung des medizinischen Instruments verbindbar ist.

10. Einführhilfe nach Anspruch 9 **gekennzeichnet durch** eine Arretiervorrichtung zur Arretierung der aktuellen Betätigungsposition der Handhabe und damit der Position des medizinischen Instruments.

11. Einführhilfe nach einem der Ansprüche 9 und 10 **gekennzeichnet durch** eine Nullpunktseinstellung zur Einstellung einer Ausgangslage des medizinischen Instruments in einer Ausgangsstellung der Handhabe.

12. Einführhilfe nach einem der Ansprüche 1 bis 11, **dadurch gekennzeichnet, dass** die einem Drehrad oder -griff aufweist, der mit einem in der Einführhilfe liegenden oder an der Einführhilfe befestigten medizinischen Instrument für eine Rotation des medizinischen Instruments verbindbar ist.

13. Einführhilfe nach Anspruch 12 **gekennzeichnet durch** eine Arretiervorrichtung zur Arretierung der aktuellen Betätigungsposition der Handhabe und damit der Ausrichtung des medizinischen Instruments.

14. Einführhilfe nach einem der Ansprüche 12 und 13 **gekennzeichnet durch** eine Nullpunktseinstellung zur Einstellung einer Ausgangsausrichtung des medizinischen Instruments in einer Ausgangstellung der Handhabe.

## Claims

1. An insertion aid for medical instruments, comprising:
a shaft (5) having a distal end (7), at which a bending part is provided having structures (3) which promote a bending in at least one preferred direction (81) different from the shaft (5); and a proximal end (6) at which a handle is provided for manually actuating the bending part for its bending in a certain angle; and a tension element (4) for actuating the bending part,
wherein the shaft (5) consists, between the handle and the bending part, of an outer tube element (2) protruding up to the distal end (7) of the shaft (5), and an inner tube element (1) guided within the outer tube element (2) and protruding beyond the outer tube element (2) in the region of the distal end (7), and the inner tube element (1) having at its distal end the bending part, at which distal end the tension element (4) is connected in a decentralized manner, **characterized in that** the tension part (4) is hinged at the distal end of the outer tube element.

2. The insertion aid according to claim 1, **characterized in that** the structures of the bending part consist of a plurality of longitudinally spaced outer notches or gaps in a wall of the inner tube element.

3. The insertion aid according to claim 1, **characterized in that** the structures are formed by a plurality of longitudinally spaced tube elements or links, which are connected to each other and to the inner tube element by hinges so as to form a continuous chain of tube elements or links.

4. The insertion aid according to one of the preceding claims, **characterized in that** the tension element is guided back through guiding bores provided in the tube elements or links to the distal end of the other outer tube element for transmitting a tensile force and/or a compressive force to the distal end of the bending part.

5. The insertion aid according to claim 1, **characterized in that** one of the handling device and the shaft includes a locking for maintaining a certain angular position of the bending part.

6. The insertion aid according to one of the claims 1 to 5, with first and second trigger guards or trigger grips, wherein one end of each of the first and second trigger guards or trigger grips is connectable to the distal end of a corresponding one of the inner and outer tube elements for a relative displacement of the inner and outer tube elements in an axial direction.

7. The insertion aid according to claim 6, **characterized by** a locking device for locking a current actuating position of the handle to maintain the angular position of the bending part.

8. The insertion aid according to one of the claims 6 and 7, **characterized by** a zero point adjustment for adjusting an initial relative position of the two tube elements in an initial position of the handle.

9. The insertion aid according to one of the claims 1 to 8, **characterized in that** the handle comprises a trigger guard or trigger grip which is connectable to a medical instrument that is one of assembled with or provided in the insertion aid for an axial displacement of the medical instrument.

10. The insertion aid according to claim 9, **characterized by** a locking device for locking the current actuating position of the handle to maintain the position of the medical instrument.

11. The insertion aid according to one of the claims 9 and 10, **characterized by** a zero point adjustment for adjusting an initial position of the medical instrument in an initial position of the handle.

12. The insertion aid according to one of the claims 1 to 11, **characterized in that** it has a turning wheel or turning grip which is connectable to a medical instrument that is provided in or assembled with the insertion aid for rotation of the medical instrument.

13. The insertion aid according to claim 12, **characterized by** a locking device for locking the current actuating position of the handle to maintain the orientation of the medical instrument.

14. The insertion aid according to one of the claims 12 and 13, **characterized by** a zero point adjustment for adjusting an initial orientation of the medical instrument in an initial position of the handle.

## Revendications

1. Auxiliaire d'insertion pour instruments médicaux, composé d'une tige (5) avec une extrémité distale (7) où est disposée une pièce de flexion comportant des structures (3) favorisant une flexion dans au moins une direction prédominante (81), à la différence de la tige (5), avec une extrémité proximale (6) où est disposée une poignée pour la commande manuelle de la pièce de flexion, pour sa flexion suivant un angle défini, et avec un élément de traction (4) pour l'actionnement de la pièce de flexion, où, entre la poignée et la pièce de flexion, la tige (5) se compose d'un élément tubulaire extérieur (2) qui s'étend jusqu'au niveau de l'extrémité distale (7) de la tige (5), et d'un élément tubulaire intérieur (1) guidé dans l'élément tubulaire extérieur (2) et dépassant de l'élément tubulaire extérieur (2) au niveau de l'extrémité distale (7), et où l'élément tubulaire intérieur (1) présente à son extrémité distale la pièce de flexion à l'extrémité distale duquel l'élément de traction (4) est fixé de manière décentrée, **caractérisé en ce que** l'élément de traction (4) est articulé à l'extrémité distale de l'élément tubulaire extérieur.

2. Auxiliaire d'insertion selon la revendication 1, **caractérisé en ce que** les structures de la pièce de flexion consistent en une pluralité d'encoches extérieures ou de fentes dans la paroi du premier élément tubulaire, espacées longitudinalement entre elles.

3. Auxiliaire d'insertion selon la revendication 1, **caractérisé en ce que** les structures sont formées par une pluralité de pièces tubulaires ou d'éléments espacés longitudinalement entre eux, et reliés entre eux et au premier élément tubulaire par des charnières de manière à constituer une chaîne continue de pièces tubulaires ou d'éléments.

4. Auxiliaire d'insertion selon l'une des revendications précédentes, **caractérisé en ce que** l'élément de traction est repassé par des alésages de guidage ménagés au travers des pièces tubulaires ou éléments vers l'extrémité distale de l'autre élément tubulaire pour une transmission de force de traction et/ou de pression à l'extrémité distale de la pièce de flexion.

5. Auxiliaire d'insertion selon la revendication 1, **caractérisé en ce que** la poignée ou la tige présentent une fixation permettant de conserver une position angulaire définie pour la pièce de flexion.

6. Auxiliaire d'insertion selon l'une des revendications 1 à 5, avec deux étriers ou manettes de traction, qui à leurs premières extrémités peuvent être reliés à une des extrémités distales de l'élément tubulaire intérieur et de l'élément tubulaire extérieur pour un déplacement relatif des deux éléments tubulaires en direction axiale.

7. Auxiliaire d'insertion selon la revendication 6, **caractérisé par** un dispositif de fixation pour la fixation de la position actuelle d'actionnement de la poignée et donc de la position angulaire de la pièce de flexion.

8. Auxiliaire d'insertion selon la revendication 6 ou la revendication 7, **caractérisé par** un réglage de point zéro pour le réglage d'une position initiale relative des deux éléments tubulaires dans une position initiale de la poignée.

9. Auxiliaire d'insertion selon l'une des revendications 1 à 8, **caractérisé en ce que** celui-ci comporte un étrier ou une manette de traction pouvant être relié à un instrument médical logé dans l'auxiliaire d'insertion ou fixé sur l'auxiliaire d'insertion, pour un déplacement axial dudit instrument médical.

10. Auxiliaire d'insertion selon la revendication 9, **caractérisé par** un dispositif de fixation pour la fixation de la position actuelle d'actionnement de la poignée et donc de la position de l'instrument médical.

11. Auxiliaire d'insertion selon la revendication 9 ou la revendication 10, **caractérisé par** un réglage de point zéro pour le réglage d'une position initiale de l'instrument médical dans une position initiale de la poignée.

12. Auxiliaire d'insertion selon l'une des revendications 1 à 11, **caractérisé en ce que** celui-ci comporte un volant ou une manette tournante pouvant être relié à un instrument médical logé dans l'auxiliaire d'insertion ou fixé sur l'auxiliaire d'insertion, pour une rotation dudit instrument médical.

13. Auxiliaire d'insertion selon la revendication 12, **caractérisé par** un dispositif de fixation pour la fixation de la position actuelle d'actionnement de la poignée et donc de l'alignement de l'instrument médical.

14. Auxiliaire d'insertion selon la revendication 12 ou la revendication 13, **caractérisé par** un réglage de point zéro pour le réglage d'un alignement initial de l'instrument médical dans une position initiale de la poignée.
